(19) Europäisches Patentamt
European Patent Office
Office européen des brevets

(11) Veröffentlichungsnummer: **0 154 793**
**B1**

(12) **EUROPÄISCHE PATENTSCHRIFT**

(45) Veröffentlichungstag der Patentschrift: **31.08.88**

(21) Anmeldenummer: **85100970.4**

(22) Anmeldetag: **31.01.85**

(51) Int. Cl.⁴: **C 07 D 498/04,** A 61 K 31/535 // (C07D498/04, 265:00, 205:00)

(54) **1-Oxadethiacephalosporinderivate sowie Verfahren zu ihrer Herstellung.**

(30) Priorität: **11.02.84 DE 3404906**

(43) Veröffentlichungstag der Anmeldung: **18.09.85 Patentblatt 85/38**

(45) Bekanntmachung des Hinweises auf die Patenterteilung: **31.08.88 Patentblatt 88/35**

(84) Benannte Vertragsstaaten: **AT BE CH DE FR GB IT LI NL SE**

(56) Entgegenhaltungen:
**EP-A-0 064 740**
**EP-A-0 074 645**
**EP-A-0 075 805**
**EP-A-0 098 609**
**DE-A-2 806 457**
**DE-A-2 943 437**
**GB-A-2 116 180**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Hartwig, Wolfgang, Dr., Pahlkestrasse 3, D-5600 Wuppertal 1 (DE)**
Erfinder: **Häbich, Dieter, Dr., Krummacherstrasse 82, D-5600 Wuppertal 1 (DE)**
Erfinder: **Metzger, Karl Georg, Dr., Pahlkestrasse 75, D-5600 Wuppertal 1 (DE)**
Erfinder: **Zeiler, Hans- Joachim, Dr., Elsbeekerstrasse 46, D-5620 Velbert 15 (DE)**

**EP 0 154 793 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die Erfindung stellt neue 1-Oxadethiacephalosporinderivate zur Verfügung, die in 3-Stellung des Cephemrings durch bestimmte Ammoniumreste substituiert sind.

Die EP-A-0 064 740 betrifft Cephalosporinderivate der allgemeinen Formel

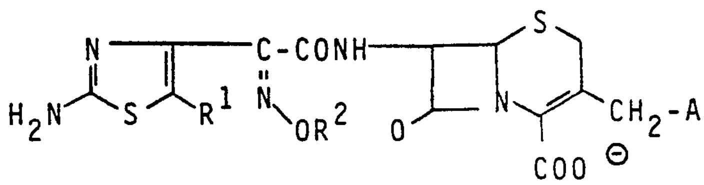

gegen bakterielle Infektionen wirksame pharmazeutische Präparate, die solche Cephemderivate enthalten, Verfahren zur Herstellung der Cephemderivate und der pharmazeutischen Präparate, Verwendung der Cephemderivate zur Bekämpfung bakterieller Infektionen und ihre Ausgangsverbindungen.

In dieser allgemeinen Formel bedeutet A einen Pyridiniumrest, der gegebenenfalls substituiert sein kann.

Es wurde gefunden, daß sich entsprechende Oxadethiacephalosporine durch verbesserte antibakterielle Wirksamkeit auszeichnen.

Die erfindungsgemäßen 1-Oxadethiacephalosporinderivate entsprechen der allgemeinen Formel 1,

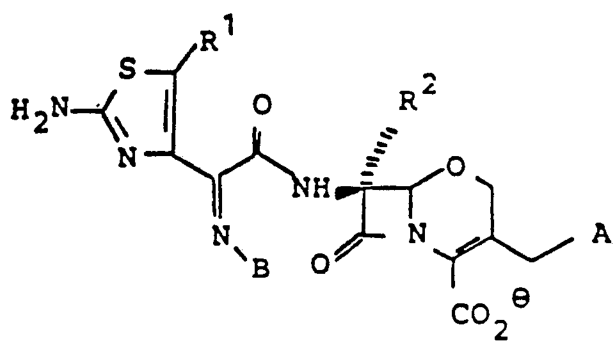

(1)

in welcher

$R^1$ Wasserstoff, Fluor, Chlor oder Brom,

$R^2$ Wasserstoff oder Methoxy bedeutet,

B eine Alkoxygruppe O-$R^3$ bedeutet, in der

$R^3$ für $C_1$-$C_6$-Alkyl oder für einen Rest $-(CH_2)_n(Z)_mR^4$ steht, in dem

$R^4$ eine Gruppe $CO_2R^5$ darstellt, in der

$R^5$ Wasserstoff, $C_1$-$C_4$-Alkyl, $CH_2OC_1$-$C_4$-Alkyl oder ein Äquivalent eines Alkalimetalls, Erdalkalimetalls, Ammonium oder einer organischen Aminbase bedeutet,

oder eine Nitrilgruppe oder Carbamoylgruppe bedeutet, und

n, m jeweils 0 oder 1 bedeutet oder

B für

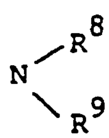 , 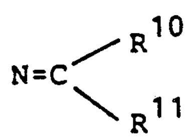 , 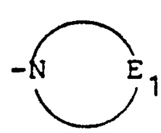 oder 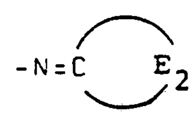 steht,

wobei

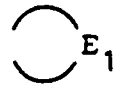

$-(CO)-(NR^{12})-(CH_2)_{n'}-$, $-(CS)-(NR^{12})-(CH_2)_{n'}-$

$-(CH_2)_{m'}-R^{13}-(CH_2)_{m'}-$

$-(CO)_{m'}-(CH_2)_p-$

$-(CO)_{2-m'}-(CH_2)_{n'}-(CO)_{m'}$

-CO-CH=CH-CO-

$-CO-O-(CH_2)_q-$

$-CO-S-(CH_2)_q-$

$-CO-NR^{12}-CO(CH_2)_{m'}-$

$-SO_2-(CH_2)_p-$

$-CO-(CH_2)_q-SO_2-$

$-CO-NR^{12}-N=CH-$

$-(CH=CH)_2-$

-CO-NH-N=CH-CO-

oder

$CO-(CH=CH)_2$

bedeutet, wobei
$R^{12}$ entweder die gleiche Bedeutung haben kann wie $R^8$ oder

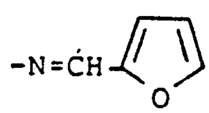

oder $-NHR^8$ bedeutet,
$R^{13}$ für O, S, SO, $SO_2$ oder $NR^{14}$ steht, wobei
$R^{14}$ Wasserstoff, Methyl, Ethyl, Cyclopropyl, Methylsulfonyl oder Furylidenamino bedeutet und
m' 1 oder 2
n' 2 oder 3,
p 3, 4 oder 5 und
q 2, 3 oder 4 ist,
$E_2$ für einen organischen Rest, der mit dem Kohlenstoffatom einen 4- bis 7-gliedrigen carbocyclischen oder heterocyclischen Ring bildet, der 1 oder 2 Sauerstoff-, Schwefel- oder Stickstoffatome enthalten kann,
$R^8$, $R^9$, $R^{10}$ und $R^{11}$ unabhängig voneinander für $C_1$- bis $C_4$-Alkyl, Benzyl, Phenylethyl, Cyclohexyl oder Phenyl oder einen heterocyclischen Rest wie

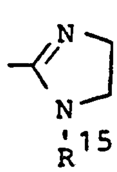 , 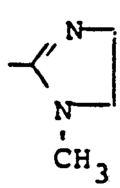 , 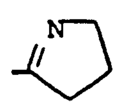 , 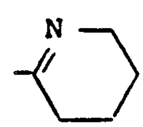 ,

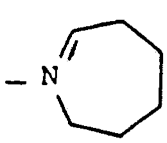 , 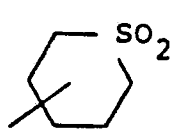 , 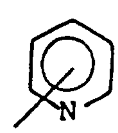 , 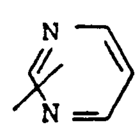 ,

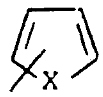 , 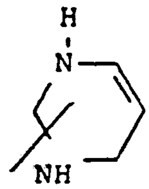 , 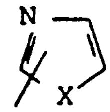 , 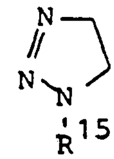 ,

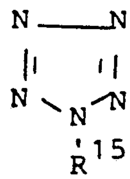 , 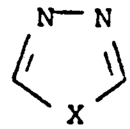 ,

stehen, wobei

$R^{15}$ für Wasserstoff oder $C_1$-$C_4$-Alkyl, Hydroxyethyl oder $C_1$-$C_4$-Alkoxyethyl und
X für S, O, SO, $SO_2$ steht,

Z eine Gruppe 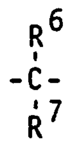

bedeutet, wobei

$R^6$ und $R^7$ gleich oder verschieden sein können und Wasserstoff, eine $C_1$-$C_4$-Alkylgruppe bedeuten, oder zusammen mit dem Kohlenstoff, an das sie gebunden sind, eine Methylen- oder eine $C_3$-$C_7$-Cycloalkylidengruppe bilden,

A einen Pyridiniumrest 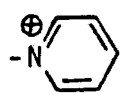

bedeutet, der entweder unsubstituiert ist oder ein- oder mehrfach, gleich oder verschieden substituiert ist,
durch $C_1$-$C_6$-Alkyl,
durch Cyano-$C_1$-$C_3$-alkyl, Epoxy-$C_2$-$C_6$-alkyl, Trifluormethyl oder Pentafluorethyl,
durch Hydroximino-methyl oder $C_1$-$C_4$-Alkoximinomethyl,
durch $C_2$-$C_6$-Alkenyl,
durch $C_2$-$C_6$-Alkinyl,
durch $C_3$-$C_7$-Cycloalkyl oder $C_3$-$C_7$-Cycloalkyl-methyl,
durch $C_4$-$C_7$-Cycloalkenyl,
durch $C_1$-$C_6$-Alkoxy,
durch Epoxy-$C_2$-$C_6$-alkoxy,
durch $C_2$-$C_6$-Alkenyloxy oder $C_2$-$C_6$-Alkinyloxy,
durch Phenoxy oder Heteroaryloxy,
durch Amino,
durch Cyano, Hydroxy oder Mercapto,
durch $C_1$-$C_6$-Alkylthio, $C_1$-$C_6$-Alkylsulfinyl oder $C_1$-$C_6$-Alkylsulfonyl,
durch $C_2$-$C_6$-Alkenylthio, $C_2$-$C_6$-Alkenylsulfinyl oder $C_2$-$C_6$-Alkenylsulfonyl,
durch Phenyl, Benzyl oder Heteroaryl,
durch Formyl,
durch $C_1$-$C_6$-Alkylcarbonyl,
durch Benzoyl,
durch $C_1$-$C_6$-Alkylcarbonylamino,
durch Carboxy oder $C_1$-$C_6$-Alkoxycarbonyl,
durch Sulfamoyl und an den ein oder zwei 3- bis 7-gliedrige Ringe ankondensiert sein können, die jeweils bis zu zwei Doppelbindungen enthalten können und auch aromatisch oder heteroaromatisch sein können.

Die vorliegende Erfindung ist insbesondere auf Verbindungen gerichtet, in denen $R^1$, $R^2$ und B die vorstehenden Bedeutungen haben und

A einen Pyridiniumrest 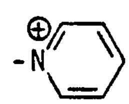

bedeutet, der entweder unsubstituiert ist, oder ein- oder mehrfach, bevorzugt 1- bis 3-fach, gleich oder verschieden substituiert ist

durch $C_1$-$C_6$-Alkyl, das ein- oder mehrfach substituiert ist,

durch Hydroxy, Carboxy, $C_1$-$C_6$-Alkyloxycarbonyl, Formyl oder $C_1$-$C_6$-Alkylcarbonyl, Carbamoyl, N-Hydroxy-carbamoyl, Sulfo, $C_1$-$C_6$-Alkyloxy, Hydroxy-$C_1$-$C_6$-Alkyloxy, $C_1$-$C_6$-Alkylthio, $C_1$-$C_6$-Alkylsulfinyl, $C_1$-$C_6$-Alkylsulfonyl, $C_2$-$C_6$-Alkenyloxy, $C_2$-$C_6$-Alkenylthio, $C_2$-$C_6$-Alkenylsulfinyl oder $C_2$-$C_6$-Alkenylsulfonyl,

durch Cyano-$C_1$-$C_3$-alkyl, Epoxy-$C_2$-$C_6$-alkyl, Hydroxyiminomethyl, $C_1$-$C_4$-Alkoxyiminomethyl, Trifluomethyl oder Pentafluorethyl,

durch $C_2$-$C_6$-Alkenyl, das durch Hydroxy suibstituiert sein kann, durch $C_2$-$C_6$-Alkinyl, durch $C_3$-$C_7$-Cycloalkyl oder $C_3$-$C_7$-Cycloalkylmethyl, wobei in beiden Substituenten der Ring auch substituiert sein kann durch Hydroxy, Halogen, Carboxy, $C_1$-$C_6$-Alkyloxycarbonyl oder Cyano,

durch $C_4$-$C_7$-Cycloalkenyl,

durch $C_1$-$C_6$-Alkoxy, das durch Hydroxy, Carboxy oder $C_1$-$C_6$-Alkyloxycarbonyl substituiert sein kann,

durch Epoxy-$C_2$-$C_6$-alkoxy,

durch $C_2$-$C_6$-Alkenyloxy oder $C_2$-$C_6$-Alkinyloxy,

durch Phenoxy,

durch Amino, das ein- oder zweifach, gleich oder verschieden substituiert sein kann durch $C_1$-$C_6$-Alkyl, Formyl, $C_1$-$C_6$-Alkylcarbonyl, $C_1$-$C_6$-Alkoxycarbonyl, Carbamoyl, $C_1$-$C_6$-Alkylsulfonyl,

durch Cyano, Hydroxy oder Mercapto,

durch $C_1$-$C_6$-Alkylthio, $C_1$-$C_6$-Alkylsulfinyl oder $C_1$-$C_6$-Alkylsulfonyl, die im Alkylteil durch Hydroxy substituiert sein können,

durch Methylthio, Methylsulfinyl oder Methylsulfonyl, die im Methylteil substituiert sind durch Carboxy oder $C_1$-$C_6$-Alkyloxycarbonyl,

durch $C_2$-$C_6$-Alkenylthio, $C_2$-$C_6$-Alkenylsulfinyl oder $C_2$-$C_6$-Alkenylsulfonyl,

durch Phenyl oder Benzyl, die auch durch Halogen substituiert sein können,

durch Formyl,

durch $C_1$-$C_6$-Alkylcarbonyl, das auch durch Hydroxy substituiert sein kann,

durch Benzoyl, $C_1$-$C_6$-Alkylcarbonylamino,

durch Carboxy oder $C_1$-$C_6$-Alkoxycarbonyl,

durch Sulfamoyl, das am Stickstoff einmal substituiert sein kann durch $C_1$-$C_6$-Alkylaminocarbonyl, und an den ein 3- bis 7-gliedriger, bevorzugt 5- oder 6-gliedriger Ring ankondensiert sein kann, der bis zu zwei Heteroatome aus der Gruppe O, N und S, und bis zu zwei Doppelbindungen enthalten kann und auch aromatisch oder heteroaromatisch sein kann und der durch folgende Substituenten ein- oder mehrfach, vorzugsweise jedoch einfach, substituiert sein kann,

durch $C_1$-$C_6$-Alkyl, $C_3$-$C_7$-Cycloalkyl, $C_1$-$C_4$-Alkoxy, $C_1$-$C_4$-Hydroxyalkyl, Halogen, Hydroxy, Oxo, Hydroximino, Exomethylen, Carboxy, $C_1$-$C_6$-Alkyloxycarbonyl, Cyano, Carbamoyl, Sulfamoyl, Amino, $C_1$-$C_4$-Alkylamino oder $C_1$-$C_4$-Dialkylamino.

Als ganz besonders bevorzugt kommen Verbindungen der Formel I in Betracht in der

$R^1$ und $R^2$ die voranstehende Bedeutung haben

A ein Pyridiniumrest ist, der entweder unsubstituiert ist oder ein oder mehrfach, vorzugsweise 1- bis 3-fach, insbesondere 1- bis 2-fach substituiert ist, beispielsweise durch Hydroxy-$C_1$-$C_4$-alkyl, wie insbesondere Hydroxymethyl, Hydroxyethyl, Hydroxypropyl, Hydroxyisopropyl, Hydroxybutyl, Hydroxy-sec.-butyl oder Hydroxy-tert.-butyl, und wobei z. B. auch zwei oder drei Hydroxylgruppen am Alkylrest stehen können,

Carboxy-$C_1$-$C_4$-alkyl, wie insbesondere Carboxymethyl und Carboxyethyl,

$C_1$-$C_4$-Alkyloxycarbonyl-$C_1$-$C_4$-alkyl, wie insbesondere Methyloxycarbonylmethyl, Ethyloxycarbonylmethyl, Methyloxycarbonylethyl,

$C_1$-$C_4$-Alkylcarbonyl-$C_1$-$C_4$-alkyl, wie insbesondere Methylcarbonylmethyl, Ethylcarbonylmethyl, Methylcarbonylethyl und Ethylcarbonylethyl, deren beide Alkylgruppen auch noch durch Hydroxy substituiert sein können,

durch Carbamoyl substituiertes $C_1$-$C_4$-Alkyl, wie insbesondere Carbamoylmethyl und Carbamoylethyl, die am Stickstoff auch noch durch Hydroxy substituiert sein können, wie insbesondere N-Hydroxy-carbamoylmethyl,

Sulfo-$C_1$-$C_4$-alkyl, wie insbesondere Sulfoethyl oder 1-Hydroxy-1-sulfomethyl,

$C_1$-$C_4$-Alkyloxy-$C_1$-$C_4$-alkyl, wie insbesondere Methyloxymethyl, Ethyloxymethyl, Propyloxymethyl, Isopropyloxymethyl, Methyloxyethyl, Ethyloxyethyl, Methyloxypropyl und Methyloxyisopropyl, die auch durch Hydroxy substituiert sein können, wie insbesondere Hydroxyethyloxymethyl und Hydroxyethyloxyethyl,

$C_1$-$C_4$-Alkylthio-$C_1$-$C_4$-alkyl, wie insbesondere Methylthiomethyl, Ethylthiomethyl, Methylthioethyl und Ethylthioethyl,

$C_1$-$C_4$-Alkylsulfinyl-$C_1$-$C_4$-alkyl, wie insbesondere Methylsulfinylmethyl, Ethylsulfinylmethyl, Methylsulfinylethyl und Ethylsulfinylethyl,

$C_1$-$C_4$-Alkylsulfonyl-$C_1$-$C_4$-alkyl, wie insbesondere Methylsulfonylmethyl, Ethylsulfonylmethyl, Methylsulfonylethyl und Ethylsulfonylethyl,

$C_3$-Alkenyloxy-$C_1$-$C_4$-alkyl, wie insbesondere Allyloxymethyl und Allyloxyethyl,

$C_3$-Alkenylthio-$C_1$-$C_4$-alkyl, wie insbesondere Allylthiomethyl,

$C_3$-Alkenylsulfinyl-$C_1$-$C_4$-alkyl, wie insbesondere Allylsulfinylmethyl,

$C_3$-Alkenylsulfonyl-$C_1$-$C_4$-Alkyl, wie insbesondere Allylsulfonylmethyl,
Cyano-$C_1$-$C_3$-alkyl, wie insbesondere Cyanomethyl und Cyanoethyl,
Epoxy-$C_2$-$C_3$-alkyl, wie insbesondere Epoxyethyl und Epoxypropyl,
Trifluormethyl, Hydroximinomethyl und $C_1$-$C_3$-Alkyl-oximinomethyl, wie insbesondere Methoximinomethyl,
$C_3$-$C_4$-Alkenyl, wie insbesondere Allyl, 2-Methylallyl und Buten-3-yl, die auch noch durch Hydroxy substituiert sein können, wie insbesondere Hydroxyallyl und Hydroxybutenyl,
$C_3$-Alkinyl, wie insbesondere Propargyl,
$C_3$-$C_6$-Cycloalkyl und $C_3$-$C_6$-Cycloalkyl-methyl, wie insbesondere Cyclopropyl, Cyclobutyl, Cyclopentyl, Cyclohexyl und Cyclopentylmethyl, wobei die Ringe auch substituiert sein können, z. B. durch Hydroxy, wie insbesondere 1-Hydroxy-1-cyclopentyl und 1-Hydroxy-1-cyclohexyl, oder durch Halogen, vorzugsweise Chlor, durch Carboxy, $C_1$-$C_4$-Alkoxycarbonyl oder Cyano,
$C_5$-$C_6$-Cycloalkenyl, wie insbesondere Cyclopenten-1-yl und Cyclohexen-1-yl,
$C_1$-$C_4$-Alkoxy, wie insbesondere Methoxy, Ethoxy, Propoxy, Isopropoxy, Butoxy, Isobutoxy und tert.-Butoxy, vorzugsweise Methoxy, wobei diese Alkoxygruppen auch noch substituiert sein können, beispielsweise durch Hydroxy, Carboxy oder $C_1$-$C_4$-Alkyloxycarbonyl, insbesondere Carboxymethyloxy und Methyloxycarbonylmethoxy,
Epoxy-$C_2$-$C_3$-alkyloxy, wie insbesondere Epoxyethoxy oder Epoxypropoxy,
$C_3$-Alkenyloxy, wie insbesondere Allyloxy,
$C_3$-Alkinyloxy, wie insbesondere Propargyloxy,
Aryloxy, wie insbesondere Phenoxy, Amino,
$C_1$-$C_5$-Alkylamino, wie insbesondere Ethylamino,
$C_1$-$C_5$-Dialkylamino, wie insbesondere Dimethylamino und Diethylamino,
$C_1$-$C_4$-Alkoxycarbonylamino, wie insbesondere Methoxycarbonylamino und Ethoxycarbonylamino,
$C_1$-$C_4$-Alkylcarbonylamino, wie insbesondere Methylcarbonylamino,
N-$C_1$-$C_4$-Alkyl- und Dialkylcarbamoylamino, wie insbesondere N-Methylcarbamoylamin, N,N-Diethylcarbamoylamino,
$C_1$-$C_4$-Alkylsulfonylamino, wie insbesondere Methyl- oder Ethylsulfonylamino,
Cyano, Hydroxy, insbesondere 3-Hydroxy, $C_1$-$C_4$-Alkylthio, wie insbesondere Methylthio, Ethylthio, Propylthio und Isopropylthio, die auch substituiert sein können durch Hydroxy, insbesondere Hydroxyethylthio,
$C_1$-$C_4$-Alkylsulfinyl, wie insbesondere Methylsulfinyl, Ethylsulfinyl, Propylsulfinyl und Isopropylsulfinyl, die auch substituiert sein können durch Hydroxy, insbesondere Hydroxyethylsulfinyl,
$C_1$-$C_4$-Alkylsulfonyl, wie Methyl- oder Ethyl- oder Propyl- oder Isopropylsulfonyl, die auch substituiert sein können durch Hydroxy, insbesondere Hydroxyethylsulfonyl,
Carboxymethylthio und $C_1$-$C_4$-Alkyloxycarbonylmethylthio, insbesondere Methyloxycarbonylmethylthio, Carboxymethyl-sulfinyl und -sulfonyl, sowie $C_1$-$C_4$-Alkyloxycarbonylmethyl-sulfinyl und -sulfonyl, insbesondere Methyloxycarbonylmethyl-sulfinyl und -sulfonyl,
$C_3$-Alkenylthio, wie Allylthio und Propen-1-ylthio,
$C_3$-Alkenylsulfinyl, wie Allylsulfinyl und Propen-1-ylsulfinyl,
$C_3$-Alkenylsulfonyl, wie Allylsulfonyl und Propen-1-ylsulfonyl,
Phenyl und Benzyl, die auch substituiert sein können, beispielsweise durch Halogen, insbesondere Chlor, wie z. B. 4-Chlorbenzyl,
2'-Thienyl und 3'-Thienyl,
Formyl und ketalisiertes Formyl, wie z. B. 1,3-Dioxolan-2-yl,
$C_1$-$C_4$-Alkylcarbonyl, insbesondere Acetyl und Propionyl, vorzugsweise Acetyl, das auch durch Hydroxy substituiert sein und auch als Ketal wie 2-Methyl-1,3-dioxolan-2-yl vorliegen kann,
Benzoyl,
$C_1$-$C_4$-Alkylcarbonylamino, insbesondere Acetylamino und Propionylamino,
Formylamino,
Carboxy, beispielsweise auch 2,3,4-Carboxy, $C_1$-$C_4$-Alkoxycarbonyl, insbesondere Methoxycarbonyl und Ethoxycarbonyl, wie z. B. auch 2,3,4-Methoxy- oder -Ethoxycarbonyl,
und an den ein 5- oder 6-gliedriger Ring ankondensiert sein kann, der bis zu zwei Heteroatome aus der Gruppe O, N und S, und bis zu zwei Doppelbindungen enthalten kann und der auch aromatisch oder heteroaromatisch sein kann. Als ankondensierte Ringe kommen insbesondere folgende Ringsysteme in Betracht.
Cyclopenteno, Dihydrocyclopenteno, Cyclohexeno, Dihydrocyclohexeno,
Benzo,
Furo, Dihydrofuro,
Pyrano, Dihydropyrano,
Thieno, Dihydrothieno,
Thiopyrano, Dihydrothiopyrano,
Pyrido, Dihydropyrido, Tetrahydropyrido,
Pyrimido, Dihydropyrimido, Tetrahydropyrimido,
Pyrazino, Dihydropyrazino, Tetrahydropyrazino,
Pyridazino, Dihydropyridazino, Tetrahydropyridazino die jeweils ein- oder mehrfach, vorzugsweise jedoch einfach, substituiert sein können, vorzugsweise durch

$C_1$-$C_4$-Alkyl, wie insbesondere Methyl, Ethyl und Isopropyl,
$C_3$-$C_6$-Cycloalkyl, wie insbesondere Cyclopropyl,
$C_1$-$C_4$-Alkoxy, wie insbesondere Methoxy und Ethoxy,
$C_1$-$C_3$-Hydroxyalkyl, wie insbesondere Hydroxymethyl, Hydroxyethyl,
Halogen, wie insbesondere Chlor und Fluor,
Hydroxy, Carboxy und Cyano,
$C_1$-$C_6$-Alkyloxycarbonyl, wie insbesondere Methoxycarbonyl und Ethoxycarbonyl,
Oxo und Hydroximino,
Carbamoyl und Sulfamoyl,
Amino,
$C_1$-$C_4$-Alkylamino, wie insbesondere Methylamino und Ethylamino,
$C_1$-$C_4$-Dialkylamino, wie insbesondere Diethylamino, und

B eine Alkoxygruppe $OR^3$ bedeutet, die vorzugsweise in "syn" Position zur Carbonamidgruppe steht, bei der $R^3$ für $C_1$-$C_5$-Alkyl, besonders für Methyl steht, oder für einen Rest $-(CH_2)_n(Z)_mR^4$ in "syn" Position,

wobei

n, m und $R^4$ die oben angegebene Bedeutung haben. Vorzugsweise steht für $-(CH_2)_n(Z)_mR^4$, $CH_2CO_2H$, $CH_2CO_2CH_3$, $CH_2CO_2C_2H_5$, $CH_2CONH_2$, $CH_2CN$, $CH(CH_3)CO_2H$, $C(CH_3)_2CO_2H$,
$CH_2$-C-$CO_2H$ sowie für
‖
$CH_2$

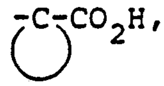

wobei

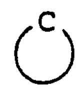

für einen carbocyclischen Ring mit 3 - 6 C-Atomen steht, der gegebenenfalls durch Alkylgruppen oder Halogen wie Chlor oder Fluor substituiert ist.

Als bevorzugte Verbindungen der vorliegenden Erfindung seien beispielhaft genannt:

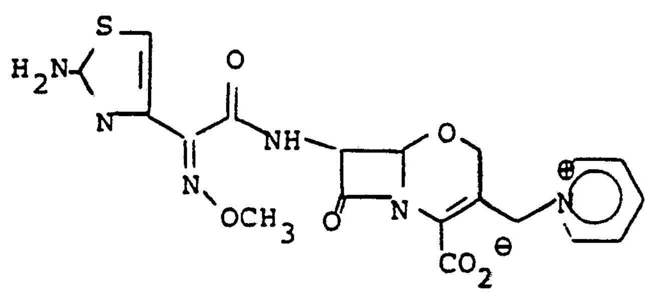

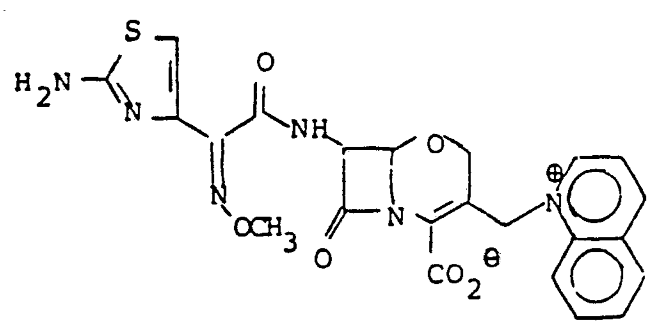

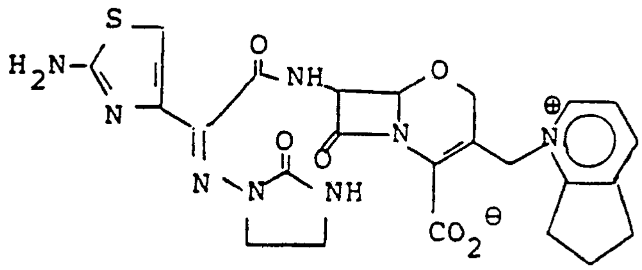

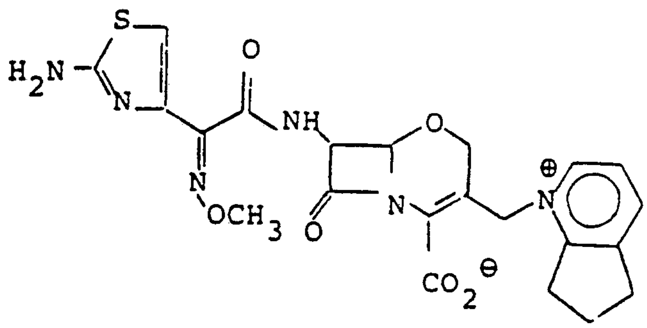

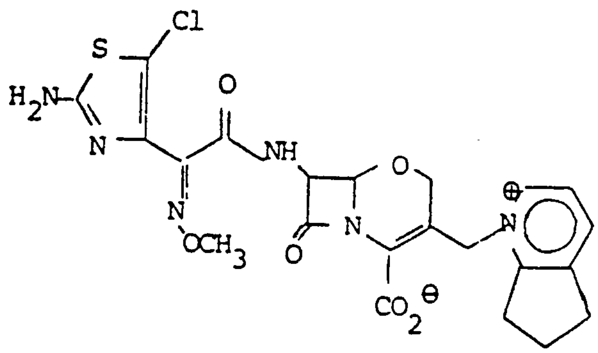

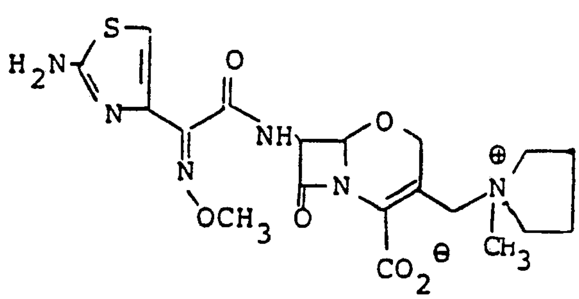

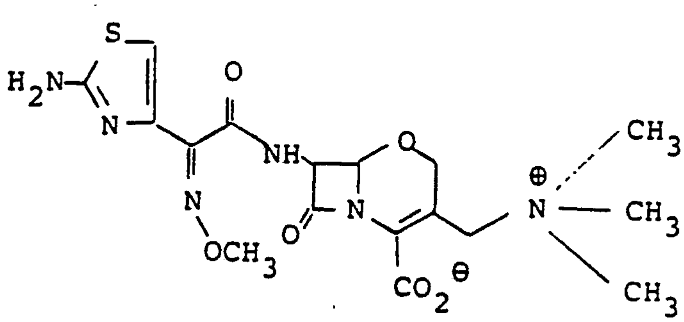

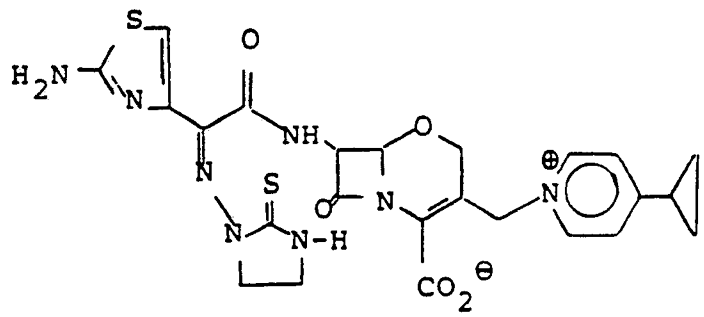

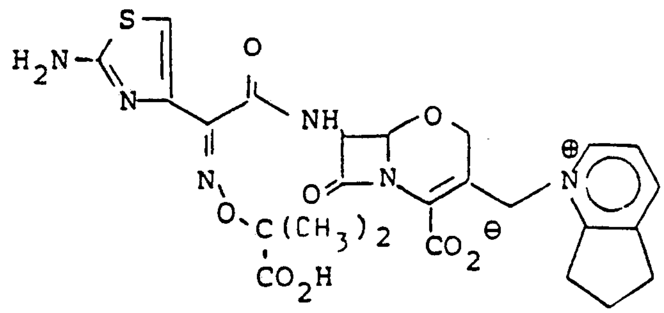

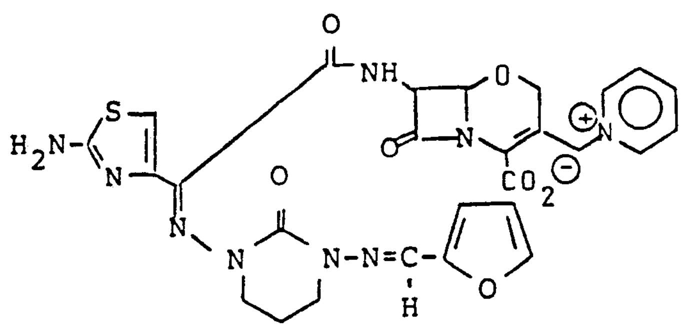

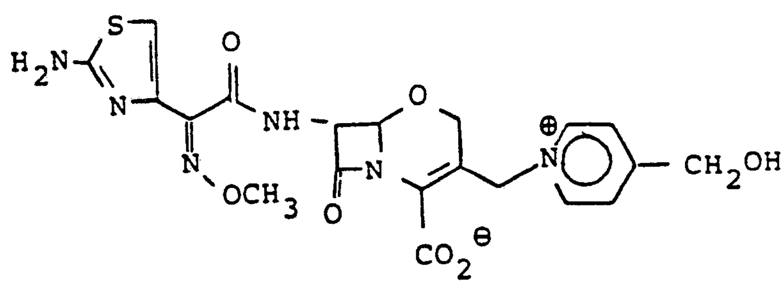

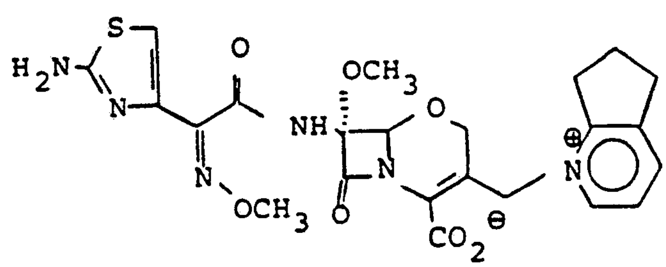

Die Verbindungen der allgemeinen Formel 1 können dadurch erhalten werden, daß man a) Verbindungen der allgemeinen Formel 2

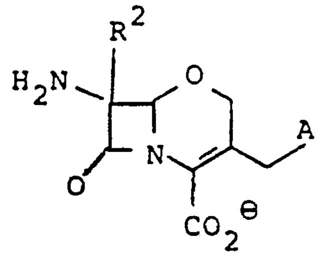

(2)

in der

A bzw. $R^2$ die oben angegebene Bedeutung haben, wobei die Aminogruppe auch in der Form eines reaktionsfähigen Derivats vorliegen kann,

mit einer 2-(2-Aminothiazol-4-yl)-2-syn-oximino-essigsäure der allgemeinen Formel 3,

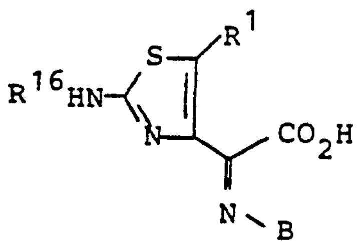

(3)

in der

B und $R^1$ die oben angegebene Bedeutung haben und

$R^{16}$ Wasserstoff oder eine Aminoschutzgruppe darstellt,

in Form eines aktivierten Derivates dieser Verbindung umsetzt und eine gegebenenfalls vorhandene Schutzgruppe abspaltet

b) eine Verbindung der allgemeinen Formel 4

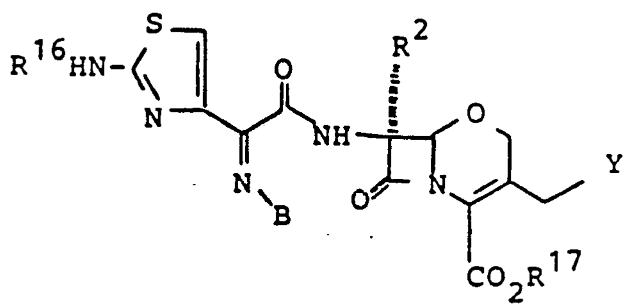

(4)

in der

$R^1$, $R^2$, $R^{16}$ und B die oben angegebene Bedeutung haben und

$R^{17}$ Wasserstoff oder einen organischen Ammoniumrest,

Y eine nucleofuge Abgangsgruppe, vorzugsweise Chlor, Brom oder Jod, Chloracetoxy, Dichloracetoxy, Mesyloxy oder Tosyloxy

bedeuten,

mit Pyridin oder einem Pyridinderivat, das einem in Formel 1 genannten Pyridiniumrest A entspricht, umsetzt, worauf, falls der Rest $R^{16}$ eine leicht abspaltbare Schutzgruppe darstellt, man diese hydrolytisch oder hydrogenolytisch abspaltet.

Die Verbindungen der allgemeinen Formel 4 können dadurch erhalten werden, daß man in Verbindungen der allgemeinen Formel 5,

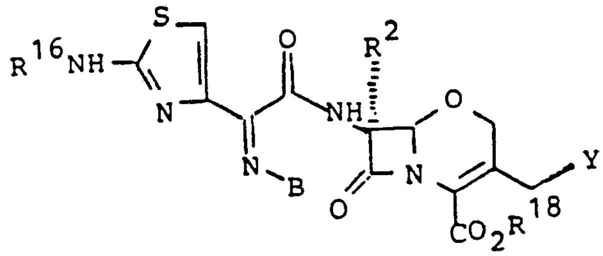

(5)

in der

$R^1$, $R^2$, $R^{16}$, B und Y die oben angegebene Bedeutung haben und

$R^{18}$ eine leicht abspaltbare Carboxylschutzgruppe wie z. B. tert.-Butyl, Benzhydryl, p-Nitrobenzyl bedeutet, die Säureschutzgruppe $R^{18}$ in Analogie zu bekannten Verfahren abspaltet.

Vorzugsweise geschieht dies im Falle des Benzhydrylesters bzw. tert.-Butylesters durch Behandeln mit Trifluoressigsäure in einem inerten organischen Verdünnungsmittel.

Die Verbindungen der allgemeinen Formel 5 können dadurch erhalten werden, daß man Verbindungen der allgemeinen Formel 3 oder ein aktiviertes Derivat dieser Verbindung mit Verbindungen der allgemeinen Formel 6,

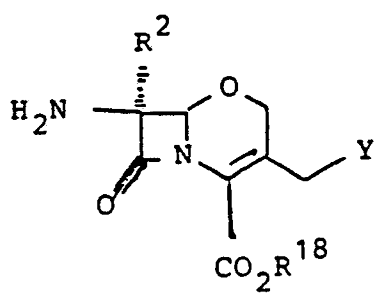

(6)

in der

$R^2$, $R^{18}$ und Y die oben angegebene Bedeutung haben

nach den in der β-Lactamchemie üblichen Methoden umsetzt.

Die Verbindungen der allgemeinen Formel 6 mit Y = Cl und OAc sind bereits bekannt (Deutsche Offenlegungsschrift 2 806 457 und US Patentschrift 432 685).

Die Verbindungen der allgemeinen Formel 6 mit X = Chloracetoxy, Dichloracetoxy, Mesyloxy oder Tosyloxy können analog den in der oben zugeführten Literatur beschriebenen Verfahren hergestellt werden, indem man den im Verlauf der Synthesesequenz auftretenden 3-Hydroxymethyl-1-oxadethiacephamcarbonsäureester mit Chloracetylchlorid, bzw. Dichloracetylchlorid, Mesylchlorid oder Tosylchlorid umsetzt.

Soll die Herstellung der Verbindungen der allgemeinen Formel 1 durch Acylierung von Verbindungen der allgemeinen Formel 2 mit Verbindungen der allgemeinen Formel 3 erfolgen, so kommen Verbindungen der allgemeinen Formel 3 in Betracht, bei denen $R^{16}$ Wasserstoff oder eine leicht abspaltbare Aminoschutzgruppe darstellt wie z. B. einen BOC-(tert.-Butoxycarbonyl), Cbz-(Carbobenzoxy), Trit-(Trityl), einen Formyl oder Chloracetylrest.

Die Acylierung der Verbindungen der allgemeinen Formel 2 bzw. 6 oder im Fall von 2 von deren Additionssalzen, beispielsweise mit Chlorwasserstoffsäure, Bromwasserstoffsäure, Salpetersäure, Phosphorsäure, oder einer organischen Säure, wie z. B. Methansulfonsäure oder p-Toluolsulfonsäure, kann mit Carbonsäuren der allgemeinen Formel 3 oder mit einem reaktionsfähigen Derivat einer solchen Säure durchgeführt werden. In manchen Fällen ist es dabei von Vorteil, die 2-Aminogruppe in den Verbindungen der allgemeinen Formel 3 vor der Umsetzung mit den vorstehend genannten Aminoschutzgruppen $R^1$ zu schützen.

Die Schutzgruppe kann nach der Acylierung in an sich bekannter Weise abgespalten werden, z. B. die Tritylgruppe oder BOC-Gruppe mittels einer Carbonsäure, wie z. B. Essigsäure, Trifluoressigsäure oder Ameisensäure.

Werden die Carbonsäuren der allgemeinen Formel 3 sowie ihre an der Aminogruppe geschützten Derivate vorzugsweise selbst als Acylierungsmittel eingesetzt, so wird zweckmäßig in Gegenwart eines Kondensationsmittels, beispielsweise eines Carbodiimids, wie beispielsweise N,N'-Dicyclohexylcarbodiimid umgesetzt.

Die Aktivierung der Carbonsäure der allgemeinen Formel 3 kann auch durch Behandlung mit bestimmten Carbonsäureamiden und beispielsweise Phosgen, Phosphorpentachlorid, Tosylchlorid, Thionylchlorid oder Oxalylchlorid erfolgen.

Als aktivierte Derivate der Carbonsäuren der allgemeinen Formel 3 eignen sich insbesondere auch Halogenide vorzugsweise Chloride, die in an sich bekanter Weise durch Behandlung mit Halogenierungsmitteln, wie z. B. Phosphorpentachlorid, Phosgen oder Thionylchlorid unter für die Cephalosporinchemie literaturbekannten, schonenden Reaktionsbedingungen erhalten werden.

Als aktivierte Derivate der Carbonsäuren der allgemeinen Formel 3 eignen sich ferner die Anhydride und gemischten Anhydride, Azide und aktivierten Ester, vorzugsweise mit p-Nitrophenol, 2,4-Dinitrophenol, Methylencyanhydrin, N-Hydroxysuccinimid und N-Hydroxyphthalimid, insbesondere diejenigen mit 1-Hydroxybenzotriazol und 6-Chlor-1-hydroxybenzotriazol. Als gemischte Anhydride sind besonders geeignet solche mit niederen Alkansäuren, wie z. B. Essigsäure, und besonders bevorzugt solche mit substituierten Essigsäuren, wie z. B. Trichloressigsäure, Pivalinsäure oder Cyanessigsäure. Geeignet sind aber auch die gemischten Anhydride mit Kohlensäurehalbestern, die man beispielsweise durch Umsetzung der Carbonsäuren der Formel 3, in denen die Aminogruppe geschützt ist, mit Chlorameisensäure-benzylester, p-nitrobenzylester, -isobutylester, -ethylester oder -allylester gewinnt. Die aktivierten Derivate können als isolierte Substanzen, aber auch in situ umgesetzt werden.

Liegt in den Verbindungen der allgemeinen Formel 2 bzw. 6 die Aminogruppe in Form eines reaktionsfähigen Derivats vor, so kann es sich um ein solches handeln, wie es aus der Literatur für Amidierungen bekannt ist. So kommen beispielsweise Silylderivate in Betracht, die bei der Umsetzung von Verbindungen der allgemeinen Formel 2 bzw. 6 mit einer Silylverbindung gebildet werden, wie z. B. Trimethylchlorsilan oder Bis-(trimethylsilyl)-acetamid. Wird die Umsetzung mit einer solchen, an der Aminogruppe aktivierten Verbindung durchgeführt, so ist es zweckmäßig, die Reaktion in einem inerten Lösungsmittel, wie z. B. Methylenchlorid, Tetrahydrofuran oder Dimethylformamid durchzuführen. Setzt man die Verbindungen der allgemeinen Formel 2 ein, so arbeitet man vorzugsweise mit einem Überschuß an Bis-(trimethylsilyl)acetamid (5 - 10 Moläquivalente), wobei auch die Carboxylgruppe silyliert wird und dadurch die Lösungseigenschaften der Verbindungen der allgemeinen Formel 2 in den oben genannten Lösungsmitteln verbessert wird.

Im allgemeinen erfolgt die Umsetzung der 1-Oxadethiacepheme der allgemeinen Formel 2 bzw. 6 mit einer Carbonsäure der allgemeinen Formel 3 oder einem aktivierten Derivat derselben in Gegenwart eines inerten Lösungsmittels. Insbesondere eignen sich chlorierte Kohlenwasserstoffe, wie vorzugsweise Methylenchlorid und Chloroform; Ether, wie z. B. Diethylether, vorzugsweise Tetrahydrofuran und Dioxan; Ketone, wie vorzugsweise Aceton und Butanon; Amide, wie vorzugsweise Dimethylformamid und Dimethylacetamid oder Wasser. Es kann sich auch als vorteilhaft erweisen, Gemische der genannten Lösungsmittel zu verwenden. Dies ist oftmals dann der Fall, wenn die 1-Oxadethiacephemverbindungen der allgemeinen Formel 2 bzw. 6 mit einem in situ erzeugten aktivierten Derivat einer Carbonsäure der Formel 3 umgesetzt wird.

Die Umsetzung von Verbindungen der Formel 2 bzw. 6 mit Carbonsäuren der Formel 3 bzw. deren aktivierten Derivaten kann in einem Temperaturbereich von etwa -80 bis etwa +80°C vorzugsweise zwischen -30 und +50°C, insbesondere jedoch zwischen etwa -20°C und Raumtemperatur erfolgen.

Die Reaktionsdauer hängt von den Reaktanten, der Temperatur und dem Lösungsmittelgemisch ab und liegt normalerweise zwischen etwa 1/2 und etwa 30 Stunden.

Die Reaktion mit Säurehalogeniden kann gegebenenfalls in Gegenwart eines säurebindenden Mittels zur Bindung des freigesetzten Halogenwasserstoffs durchgeführt werden. Als solche eignen sich insbesondere tertiäre Amine, wie z. B. Triethylamin oder Dimethylanilin, anorganische Basen, wie z. B. Kaliumcarbonat oder Natriumcarbonat, Alkylenoxide, wie z. B. Propylenoxid. Auch die Anwesenheit eines Katalysators, wie z. B. von Dimethylaminopyridin kann gegebenenfalls von Vorteil sein.

Soll die Herstellung der Verbindungen der allgemeinen Formel 1 durch nucleophilen Austausch von Y in den Verbindungen der allgemeinen Formel 4 durch Pyridin oder eines der angegebenen Pyridinderivate erfolgen, so kommen als Reste Y Halogenatome, vorzugsweise Chlor aber auch Chloracetoxy, Dichloracetoxy oder Mesyloxy- oder Tosyloxy in Betracht. $R^{17}$ steht vorzugsweise für Wasserstoff.

Die Pyridinkomponente wird in Mengen zugegeben, die zwischen äquimolaren und einem bis zu 30-fachen Überschuß liegt.

Als Lösungsmittel bei diesen Umsetzungen kommen alle inerten organischen Verdünnungsmittel in Betracht. Hierzu gehören halogenierte Kohlenwasserstoffe wie Dichlormethan, Trichlormethan oder 1,2-Dichlorethan, cyclische Ether wie Tetrahydrofuran und Dioxan oder dipolare aprotische Lösungsmittel wie z. B. Dimethylformamid oder Mischungen derselben.

Die Umsetzungen erfolgen im allgemeinen bei Temperaturen zwischen -20°C und 40°C, vorzugsweise bei 0°C bis 25°C.

Bei den Umsetzungen der Verbindungen der allgemeinen Formel 5 zu den Verbindungen der allgemeinen Formel 4 kommen alle Reagenzien in Betracht, die die in der β-Lactamchemie üblicherweise verwendeten Säureschutzgruppen selektiv zu spalten vermögen. Hierzu gehören vorzugsweise Ameisensäure und wasserfreie Trifluoressigsäure. Die Umsetzung kann gegebenenfalls unter Zusatz von 1 bis 10 Äquivalenten Amisol durchgeführt werden.

Als Verdünnungsmittel kommen alle inerten organischen Lösungsmittel in Frage wie z. B. halogenierte Kohlenwasserstoffe wie Dichlormethan, Trichlormethan, Tetrachlormethan oder 1,2-Dichlorethan.

Die Umsetzungen erfolgen im allgemeinen bei Temperaturen zwischen -70°C und 40°C, vorzugsweise zwischen -10°C und 0°C.

Beim Abziehen des Lösungsmittels nach erfolgter Umsetzung 5 → 4 empfiehlt es sich, 1 - 50 Mol-Äquivalente eines aromatischen Kohlenwasserstoffes, vorzugsweise Benzol zuzusetzen.

Die Verbindungen der allgemeinen Formel 4 können als solche isoliert werden, vorzugsweise setzt man sie aber direkt nach Abdestillieren des Lösungsmittels und des Überschusses an Säure wie oben beschrieben zu den Verbindungen der allgemeinen Formel 1 um.

Verwendet man 2-(2-Aminothiazol-4-yl)-2-syn-methoximinoessigsäure und (6R, 7S)-7β-Amino-3[(2,3-cyclopenteno-1-pyridinio)methyl-8]-oxo-5-oxa-1-aza-bicyclo[4.2.0]oct-2-en-2-carboxylat als Ausgangsmaterialien, so kann der Reaktionsablauf durch das Formelschema I wiedergegeben werden:

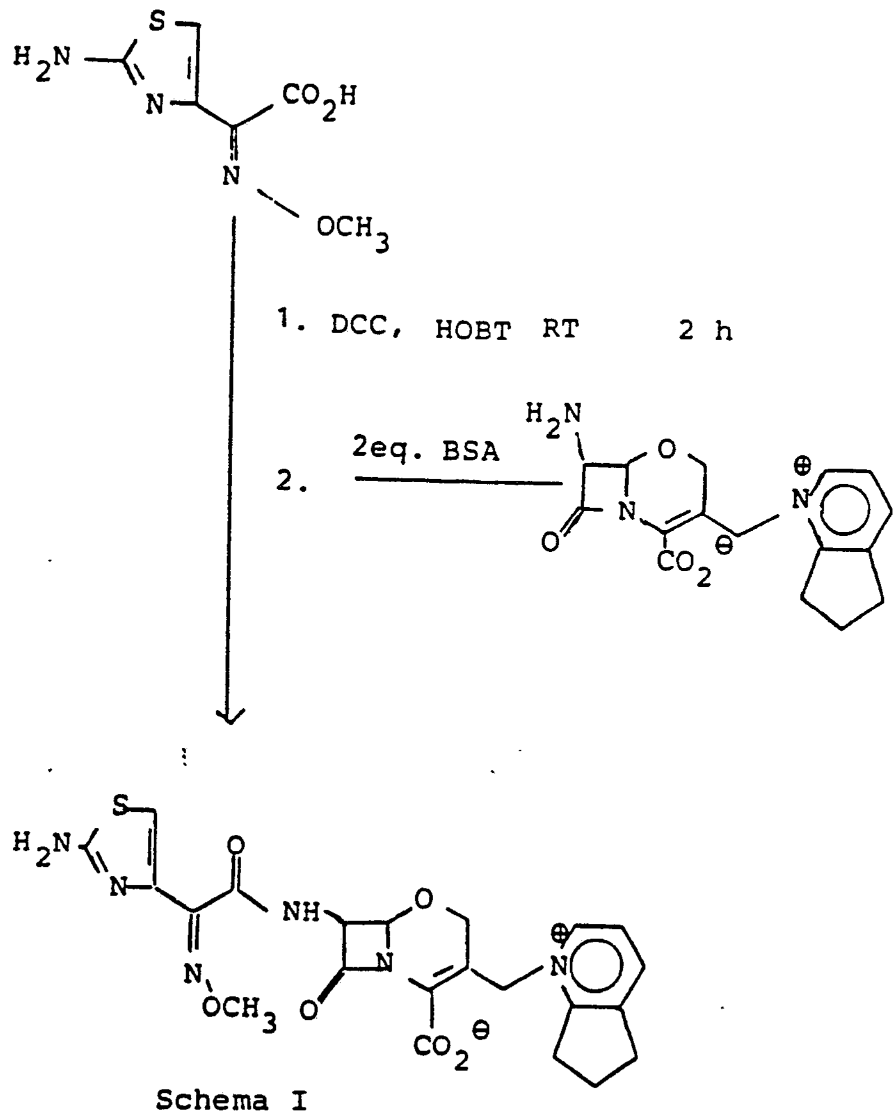


Schema I

Verwendet man (6R, 7S)-7β-Amino-3-chloromethyl-8-oxo-5-oxa-1-aza-bicyclo [4.2.0] oct-2-en-2-carbonsäurebenzhydrylester sowie 2-(2-Aminothiazol-4-yl)-2-syn-methoximinoessigsäure und Pyridin als Ausgangsmateralien so kann der Reaktionsablauf durch das Formelschema II wiedergegeben werden:

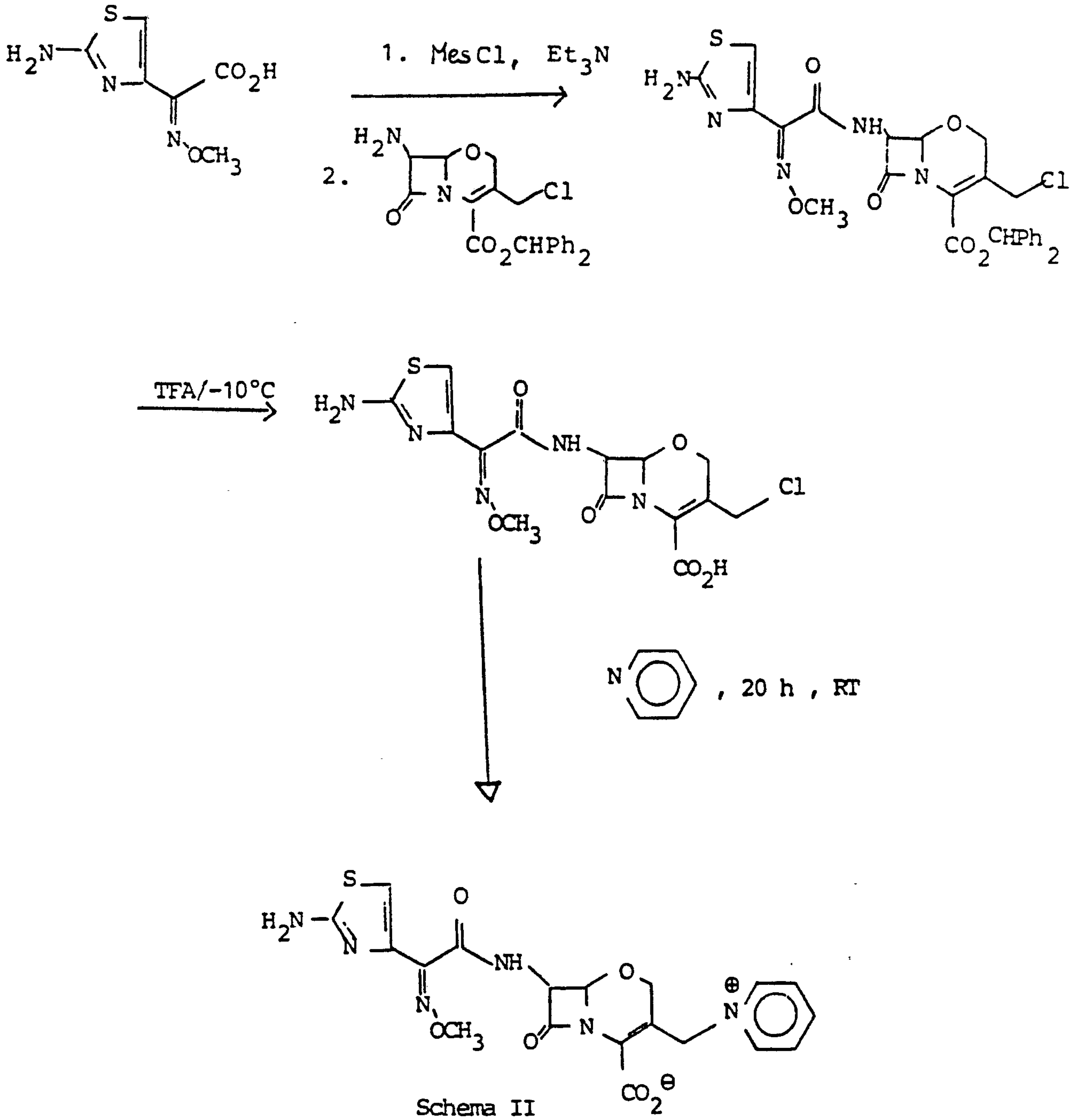


Schema II

Die erfindungsgemäßen Verbindungen weisen eine starke und breite antimikrobielle Wirksamkeit besonders gegen gram-negative und gram-positive Bakterien auf. Diese Eigenschaften ermöglichen ihre Verwendung als chemotherapeutische Wirkstoffe in der Medizin. Mit ihrer Hilfe können die durch gram-negative und gram-positive Bakterien und bakterienähnliche Mikroorganismen hervorgerufenen Erkrankungen verhindert, gebessert und/oder geheilt werden.

Besonders wirksam sind die erfindungsgemäßen Verbindungen gegen Bakterien und bakterienähnliche Mikroorganismen.

Sie sind daher besonders gut zur Prophylaxe und Chemotherapie von lokalen und systemischen Infektionen in der Human- und Tiermedizin geeignet, die durch diese Erreger hervorgerufen werden.

Beispielsweise können lokale und/oder systemische Erkrankungen behandelt und/oder verhindert werden, die durch die folgenden Erreger oder durch Mischungen der folgenden Erreger verursacht werden:

Pseudomonoadaceae, wie Pseudomonas-Bakterien, z. B. Pseudomonas aeruginosa, (PS. = Pseudomonas);

Micrococcaceae, wie Staphylokokken, z. B. Staphylococcus aureus, Staph. epidermidis, Staph. aerogenes und Gaffkya tetragena, (Staph. = Staphylococcus);

Lactobacteriaceae, wie Streptokokken, z. B. Streptococcus pyogenes, α- bzw. β-hämolysierende

Streptokokken, nicht (γ-)-hämolysierende Streptokokken, Str. viridans, Str. faecalis (Enterokokken) und Dipolococcus pneumoniae (Pneumokokken), (Str. = Streptococcus);

Enterobacteriaceae, wie Escherichiae-Bakterien der Coli-Gruppe: Escherichia-Bakterien, z. B. Escherichia coli, Enterobacter-Bakterien, z. B. E. aerogenes, E. cloacae, Klebsiella-Bakterien, z. B. K. pneumoniae, Serratia, z. B. Serratia marvescens, (E. = Enterobacter), (K. = Klebsiella), Proteae-Bakterien der Proteus-Gruppe: Proteus, z. B. Proteus vulgaris, Pr. morganii, Pr. rettgeri, Pr. mirabillis, (Pr. = Proteus);

Bacteroidaceae, wie Bacteroides-Bakterien, z. B. Bacteroides fragilis, (B. = Bacteroides).

Die obige Aufzählung von Erregern ist lediglich beispielhaft und keineswegs beschränkend aufzufassen.

Als Krankheiten, die durch die erfindungsgemäßen Verbindungen verhindert, gebessert und/oder geheilt werden können seien beispielsweise genannt:

Erkrankungen der Atemwege und des Rachenraumes;

Otitis; Pharyngitis; Pneumonie; Peritonitis; Pyelonephritis; Cystitis; Endocarditis; Systeminfektionen; Bronchitis; Arthritis; lokale Infektionen.

Zur vorliegenden Erfindung gehören pharmazeutischen Zubereitungen, die neben nichttoxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen.

Zur vorliegenden Erfindung gehören auch pharmazeutische Zubereitungen in Dosierungseinheiten. Dies bedeutet, daß die Zubereitung in Form einzelner Teile, z. B. Tabletten, Dragees, Kapseln, Pillen, Suppositorien und Ampullen vorliegen, deren Wirkstoffgehalt einem Bruchteil oder einem Vielfachen einer Einzeldosis entsprechen. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nichttoxischen, inerten pharmazeutisch geeigneten Trägerstoffen sind feste, halbfeste oder flüssige Verdünnungsmittel, Füllstoffe und Formulierungenshilfsmittel jeder Art zu verstehen.

Als bevorzugte pharmazeutische Zubereitungen seien Tabletten, Dragees, Kapseln, Pillen, Granulate, Suppositorien und Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie

(a) Füll- und Streckmittel, z. B. Stärken, Milchzucker, Rohrzucker, Glukose, Mannit und Kieselsäure,
(b) Bindemittel, z. B. Carboxymethylcellulose, Alginate, Gelatine, Polyvinylpyrrolidon,
(c) Feuchthaltemittel, z. B. Glycerin,
(d) Sprengmittel, z. B. Agar-Agar, Calciumcarbonat und Natriumcarbonat,
(e) Lösungsverzögerer, z. B. Paraffin und
(f) Resorptionsbeschleuniger, z. B. quarternäre Ammoniumverbindungen
(g) Netzmittel, z. B. Cetylalkohol, Glycerinmonostearat,
(h) Adsorptionsmittel, z. B. Kaolin und Bentonit und
(i) Gleitmittel, z. B. Talkum, Calcium-Magnesiumstearat und feste Polyethylenglykole

oder Gemische der unter (a) bis (i) aufgeführten Stoffe.

Die Tabletten, Dragees, Kapseln, Pillen und Granulate können mit den üblichen, gegebenenfalls Opakisierungsmittel enthaltenden Überzügen und Hüllen versehen sein und auch so zusammengesetzt sein, daß sie den oder die Wirkstoffe nur oder bevorzugt in einem bestimmten Teil des Intestinaltraktes gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffe auch in mikroverkapselter Form vorliegen.

Suppositorien können neben dem oder den Wirkstoffen die üblichen wasserlöslichen oder wasserunlöslichen Trägerstoffe enthalten, z. B. Polyethylenglykole, Fette, z. B. $C_{14}$-Alkohol mit $C_{16}$-Fettsäure, oder Gemische dieser Stoffe.

Zur parenteralen Applikation können die Lösungen auch in steriler und blutisotonischer Form vorliegen.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Die Wirkstoffe oder die pharmazeutischen Zubereitungen können lokal, oral, parenteral, intraperitonal und/oder rectal, vorzugsweise oral oder parenteral wie intravenös oder intramuskulär appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 1 bis etwa 1000, vorzugsweise 1 bis 200 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe, vorzugsweise in Mengen von etwa 1 bis etwa 250, insbesondere 1 bis 60 mg/kg Körpergewicht. Es

kann jedoch erforderlich sein, von den genannten Dosierungen abzuweichen, und zwar in Abhängigkeit von der Art und dem Körpergewicht des zu behandelnden Objekts, der Art und der Schwere der Erkrankungen, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der obengenannten Menge Wirkstoff auszukommen, während in anderen Fällen die oben angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Die erfindungsgemäßen Verbindungen können zum Zwecke der Erweiterung des Wirkungsspektrums mit einem anderen β-Lactamantibiotikum oder auch mit Aminoglykosidantibiotica, wie z. B. Gentamicin, Sisomicin, Kanamicin, Amikacin oder Tobramicin kombiniert werden.

Die erfindungsgemäßen Verbindungen eignen sich besonders zur Verwendung bei der Bekämpfung von Infektionskrankheiten, insbesondere bei der Bekämpfung bakterieller Infektionen.

**MHK-Tabelle**
Iso-Sensitest-Agar
Keimverd. ÜK 1 : 500
Präparate Konz.: µg/ml

| Keime | Beispiel 1 | Beispiel 2 | Beispiel 3 | Mezlocillin | Cefotaxim |
|---|---|---|---|---|---|
| E.coli T 7 | 4 | 4 | 2 | >128 | ⩽0,062 |
| E.coli A 261 | ⩽0.062 | 0,125 | ⩽0,062 | 128 | ⩽0,062 |
| E.coli Neumann | ⩽0.062 | ⩽0.062 | ⩽0.062 | 0.25 | ⩽0.062 |
| E.coli 183/58 | ⩽0.062 | 0.25 | ⩽0.062 | 4 | 0.5 |
| E.coli F 14 | 16 | 32 | 8 | >128 | <0.062 |
| E.coli C 165 | ⩽0.062 | ⩽0.062 | ⩽0.062 | 1 | ⩽0.062 |
| E.coli 4322 | ⩽0.062 | 1 | ⩽0.062 | 2 | ⩽0.062 |
| Klebs. 57 USA | 0.5 | 2 | 0.5 | >128 | ⩽0.062 |
| Klebs. 63 | ⩽0.062 | 0.125 | ⩽0.062 | 2-4 | ⩽0.062 |
| Klebs. 1852 | 1 | 2 | 0.5 | >128 | ⩽0.062 |
| Klebs. 6097 | ⩽0.062 | 0.25 | ⩽0.062 | 8 | ⩽0.062 |
| Serratia 16001 | ⩽0.062 | 0.125 | 0.5 | 2 | 0.5 |
| Serratia 16002 | 1 | 0.25 | 0.5 | 4 | 0.25 |
| Provid. 12012 | ⩽0.062 | 0.025 | ⩽0.062 | 2 | ⩽0.062 |
| Prot. mag. 932 | 1 | 4 | 0.125 | 32 | 4 |
| Prot. vulg. 9023 | 0.25 | 0.25 | ⩽0.062 | 1 | ⩽0.062 |
| Prot. vulg. 1017 | 32 | 32 | 16 | 4 | 0.25 |
| Prot. vulg. N 6 | 0.25 | 0.25 | 0.125 | 0.5 | ⩽0.062 |
| Prot. rettg. 10007 | ⩽0.062 | ⩽0.062 | ⩽0.062 | 0.05 | ⩽0.062 |
| Prot. mirab. 1235 | 0.125 | 0.125 | ⩽0.062 | 0.125 | ⩽0.062 |
| Staph. 1756 | >128 | >128 | >128 | 64 | >128 |
| Staph. 133 | 0.25 | 0.5 | 0.125 | 0.5 | 1 |
| Staph. 25022 | 0.5 | 1 | 0.25 | 1-2 | 1 |
| Staph. 25470 | >128 | >128 | >128 | 32-64 | >128 |
| Staph. E 25185 | 0.125 | 0.25 | ⩽0.062 | 1 | 0.5 |
| Strept. faec. 27101 | >128 | >128 | >128 | 64 | >128 |
| Strept. faec. 113 | 8 | 32-64 | 16 | 1 | 128 |
| Enterocoe 9790 | >128 | >128 | >128 | 2 | >128 |
| Enterocoe 27158 | 128 | 16 | 128 | 2 | 64 |
| Pseudom. F 41 | 1 | 4 | 0.5 | 32 | 16 |
| Pseudom. Walter | 2 | 2 | 1 | 32 | 16 |
| Pseudom. 7035 | 2 | 2 | 1-2 | 16 | 16 |
| Pseudom. 7451 | 2 | 16 | 4 | 64 | 64 |
| Enterob. cloacae 56US | >128 | 128 | 64 | 64 | >128 |
| Enterob. cloacae 5744 | 0.5-2 | 0.5 | ⩽0.062 | 16 | 0.125 |
| Achromob. CB 20005 | 0.25 | 0.5 | ⩽0.062 | 2 | ⩽0.062 |

**Beispiel 1**

(6R, 7S)-7-[2-(2-Aminothiazol-4-yl)-2-syn-methoxyimino-acetamido]-3-[1-pyridinomethyl]-8-oxo-5-oxa-1-aza-bicyclo[4.2.0]oct-2-en-2-carboxylat

a) Zur Lösung von 0,36 g 2-(2-Aminothiazol-4-yl)-2-syn-methoximinoessigsäure in 5 ml absolutem DMF gibt man bei Raumtemperatur 0,24 g N-Hydroxybenztriazol, kühlt auf 10°C und tropft die Lösung von 0,37 g N,N-Dicyclohexylcarbodiimid in 2 ml absolutem DMF zu. Man läßt 2 h bei Raumtemperatur rühren und gibt die Lösung von 0,7 g (6R, 7S)-7-amino-3-[1-pyridinomethyl]-8-oxo-5-oxa-1-aza-bicyclo[4.2.0]oct-2-en-2-carboxylat (2a) und 0,73 g Bis-(trimethylsilyl)-acetamid in 3 ml absolutem DMF vorgekühlt auf -10°C zu. Man läßt 4 h bei RT rühren, gibt 200 ml Aceton zu und saugt vom ausgefallenen Niederschlag ab. Man erhielt 310 mg der rohen Titelverbindung, die an Kieselgel mit Acetonitril/Wasser = 2/1 chromatographiert wurde. Abdampfen des Acetonitrils und Gefriertrocknung liefert 61 mg der reinen Titelverbindung als farbloses Lyophilisat.

Rf: ($CH_3CN/H_2O$ = 2/1) = 0,27

$^1$H-NMR (200 MHz, $D_2O$): δ = 3,85 (s; 3H, $OCH_3$); 4,27 u. 4,39 (AB-System, $J_{AB}$ = 18,5 Hz; 2H, $OCH_2$); 5,23 (d, J = 4 Hz; 2H, 6-H); 5,49 (d, J = 4 Hz; 1H, 7-H); 5,19 und 5,64 (AB-System, $J_{AB}$ = 15 Hz; 2H, $CH_2$-4N$^{\oplus}$); 6,87 (s; 1H, Thiazol-H); 7,95 (t, J = 7,5 Hz; 2H, 2,5-Pyrid.-H); 8,70 (t, J = 7,5 Hz; 1H, 4-Pyrid.-H); 8,86 (d, J = 6 Hz; 2H, 2,6-Pyrid-H).

b) Die Lösung von 0,53 g 2(-2-Aminothiazol-4-yl)-2-syn-methoximinoessigsäure (3a), und 1,38 ml Triethylamin in 5 ml abs. DMF wird unter $N_2$ auf -50°C gekühlt und mit 0,21 ml Mesylchlorid versetzt. Es wird kurz auf -25°C erwärmt, so daß sich eine klare Lösung bildet, anschließend wird wieder auf -50°C gekühlt und 30 min gerührt (Lösung 1).

In einem separaten Kolben werden 0,86 g (6R, 7S)-7-Amino-3-[-1-pyridinomethyl]-8-oxo-5-oxa-1-aza-bicyclo-[4.2.0]oct-2-en-carboxylat in 1 ml Wasser gelöst und unter Eiskühlung mit 0,3 ml Triethylamin versetzt. Zu dieser Lösung gab man unter Eiskühlung Lösung 1 zu, wobei durch sukzessive Zugabe vom Triethylamin der pH bei 8 - 9 gehalten wurde. Man rührte 30 min nach, gab 200 ml Aceton zu und saugte ab. Nach Chromatographie (Kieselgel; $CH_3CN/H_2O$ = 2/1) und Gefriertrocknung erhielt man 20 mg der Titelverbindung als farbloses Lyophilisat. [Phys. Daten identisch mit Beispiel 1a)].

**Beispiel 2**

(6R, 7S)-7-[2-(2-Aminothiazol-4-yl)-2-syn-ethoxyimino-acetamido]-3-[1-pyridinomethyl]-8-oxo-5-oxa-1-aza-bicyclo[4.2.0]oct-2-en-2-carboxylat

Man verfuhr analog wie in Beispiel 1a) beschrieben. Eingesetzt wurden 1,3 g (2a) und 0,72 g 2(-2-Aminothiazol-4-yl)-2-syn-ethoxyiminoessigsäure (3b).

Man erhielt 41 mg der Titelverbindung als farbloses Lyophilisat von Rf = 0,28

$^1$H-NMR ($D_2O$, 200 MHz): δ = 1,23 (t, J = 7,5 Hz; 3H, $CH_3$); 4,24 (q, J = 7,5 Hz; 2H, $\underline{CH_2}$-$CH_3$); 4,40 u. 4,55 (AB-System, $J_{AB}$ = 18,5 Hz; 2H, O-$CH_2$); 5,43 (d, J = 4 Hz; 1H, 6-H); 5,63 (d, J = 4 Hz; 1H, 7-H); 5,34 u. 5,79 (AB-System, $J_{AB}$ = 15 Hz; 2H, $CH_2N^{\oplus}$); 6,97 (s; 1H, Thiazol-H); 8,12 (t, J = 7,5 Hz; 2H, 3,5-Pyrid.-H); 8,62 (t; J = 7,5 Hz; 1H, 4-Pyrid.-H); 9,03 (d, J = 7 Hz; 2H, 2,6-Pyrid.-H).

**Beispiel 3**

(6R, 7S)-7-[2-(2-Aminothiazol-4-yl)-2-syn-methoximino-acetamido]-3-[(2,3-cyclopenteno-1-pyridinio)-methyl]-8-oxo-5-oxa-1-aza-bicyclo[4.2.0]oct-2-en-2-carboxylat

Man verfuhr analog wie in Beispiel 1a) beschrieben. Eingesetzt wurden 0,36 g (6R, 7S)-7-Amino-3[(2,3-cyclopenteno-1-pyridinio)-methyl]-8-oxo-5-oxa-1-aza-bicyclo[4.2.0]oct-2-en-2-carboxylat und 0,16 g (3a). Man erhielt 37 mg der Titelverbindung als farbloses Lyophilisat mit Rf = 0,29 ($CH_3CN/H_2O$ = 2/1)

$^1$H-NMR (200 MHz, $D_2O$): δ = 2,19 (hept. erk. als t, J = 7,5 Hz; 2H, β-Cyclopent.-H); 3,07 (t, J = 8 Hz; 2H, γ-Cyclopent.-H); 3,84 (s; 1H, $OCH_3$); 4,2 u. 4,34 (AB-System, $J_{AB}$ = 18,5 Hz; 2H, O-$CH_2$); 5,18 (d, J = 4 Hz; 1H, 6-H); 5,27 u. 5,37 (AB-System, $J_{AB}$ = 16 Hz; 2H, $CH_2$-N$^{\oplus}$); 5,49 (d, J = 4 Hz; 1H, 7-H); 6,86 (s; 1H, Thiazol-H); 7,62 (t, J = 7,5 Hz; 1H, 5-Pyrid.-H); 8,15 (d, J = 7,5 Hz; 1H, 4-Pyrid.-H); 8,44 (d, J = 7,5 Hz; 1H, 6-Pyrid.-H).

**Beispiel 4**

(6R, 7S)-7-[2-Aminothiazol-4-yl)-2-syn-ethoximino-acetamido]-3-[(2,3-cyclopenteno-1-pyridino)-methyl]-8-oxo-5-oxa-1-aza-bicyclo[4.2.0]oct-2-en-2-carboxylat

Man verfuhr analog Beispiel 1a). Man erhielt die Titelverbindung mit Rf = 0,28 und

$^1$H-NMR (200 MHz, $D_2O$): δ = 1,25 (t, J = 7,5 Hz; 3H, $CH_3$); 2,20 (hept. erk. als t, J = 7,5 Hz; 2H, β-Cyclopent.-H); 3,12 (t, J = 8 Hz; 2H, γ-Cyclopent.-H); 3,25 (t, J = 8 Hz; 2H, α-Cyclopent.-H); 4,28 (q, J = 7,5 Hz; 2H, $\underline{CH_2}$-$CH_3$); 4,29 u. 4,41 (AB-System, $J_{AB}$ = 18,5 Hz; 2H, $OCH_2$); 5,21 (d, J = 4 Hz; 1H, 6-H); 5,30 u. 5,39 (AB-System, $J_{AB}$ = 16 Hz; 2H, $CH_2$-N$^{\oplus}$); 5,50 (d, J = 4 Hz; 1H, 7-H); 6,90 (s; 1H, Thiazol-H); 7,64 (t, J = 7,5 Hz; 1H, 5-Pyrid.-H); 8,18 (d, J = 7,5 Hz; 1H, 4-Pyrid.-H); 8,49 (d, J = 7,5 Hz; 1H, 6-Pyrid.-H).

**Beispiel 5**

(6R, 7S)-7-[2-(2-Aminothiazol-4-yl)-2-syn-methoximino-acetamido]-3-chloromethyl-8-oxo-5-oxa-1-aza-bicyclo[4.2.0]oct-2-en-2-carbonsäurebenzhydrylester

Zur Lösung von 1,82 g (3a) und 2,2 ml Triethylamin in 10 ml DMF gab man bei -50°C unter $N_2$-Atmosphäre 0,7 ml Mesylchlorid und rührte 30 min bei -50°C, und 20 min bei -25°C nach. Anschließend gab man die Lösung von 3 g (6R, 7S)-7-Amino-3-chlormethyl-8-oxo-5-oxa-1-aza-bicyclo[4.2.0]oct-2-en-2-carbonsäurebenzylhydrylester gelöst in 2 ml THF und vorgekühlt auf -40°C zu, und rührte bei -40°C. Man ließ auf Raumtemperatur kommen, gab 200 ml Phosphatpufferlösung (pH = 7) zu und extrahierte dreimal mit je 100 ml Essigester. Die vereinigten organischen Extrakte wurden mit ges. $NaHCO_3$-Lösung und mit Wasser gewaschen, über $MgSO_4$ getrocknet und das Solvens i. Vak. abgezogen. Man erhielt 3,3 g der rohen Titelverbindung die an Kieselgel mit Toluol/Essigester = 1/1 und anschließend mit Essigester chromatographiert wurde. Man erhielt 350 mg der reinen Titelverbindung mit Rf = 0,38 (Essigester).

$^1$H-NMR (200 MHz, $CDCl_3$): δ = 4,04 (s; 3H, $OCH_3$); 4,48 u. 4,64 (AB-System, $J_{AB}$ = 17,5 Hz; 2H, $OCH_2$); 4,58 u. 4,59 (2s; 2H, $CH_2Cl$); 5,19 (d, J = 4 Hz; 1H, 6-H); 5,88 (dd, $J_1$ = 4 Hz, $J_2$ = 10 Hz; 1H, 7-H); 6,90 (s; 1H, CH(Ph)$_2$); 6,94 (S; 1H, Thiazol-H); 7,15-7,55 (m; 11H, Phenyl und NH).

**Beispiel 6**

(6R, 7S)-7-[2-(2-Aminothiazol-4-yl)-2-syn-methoximino-acetamido]-3-[(2,3-cyclopenteno-1-pyridinio)-methyl]-8-oxo-5-oxa-1-aza-bicyclo[4.2.0]oct-2-en-2-carboxylat

Zu einer auf -10°C gekühlten Lösung von 280 mg der Titelverbindung aus Beispiel 5 in 3 ml absolutem Dichlorethan und 1 ml Anisol gab man unter Stickstoffatmosphäre 1,42 ml Trifluoressigsäure. Man rührte 30 min bei -10°C, gab 5 ml Benzol zu und engte im Vakuum (Badtemp. 15°C) auf ein Viertel des Volumens ein. Man verdünnte mit 2 ml abs. THF, kühlte auf -15°C und gab 2 ml 2,3-Cyclopentenopyridin zu. Man ließ auf Raumtemperatur kommen und rührte 20 h nach. Man gab 2 ml THF zu und ließ langsam unter Rühren 50 ml Diethylether zulaufen. Der Niederschlag wurde unter Stickstoffatmosphäre abgesaugt, mit Ether gewaschen und an Kieselgel mit Acetonitril/$H_2O$ = 2/1 chromatographiert. Abdampfen des Acetonitrils und anschließende Gefriertrocknung lieferte 21 mg der Titelverbindung (identisch mit dem nach Beispiel 1a) hergestellen Präparat).

**Patentansprüche** für die Vertragsstaaten: (BE, DE, FR, IT, NL, CH, SE, GB, LI)

1. 1-Oxadethiacephalosporinderivate der allgemeinen Formel 1,

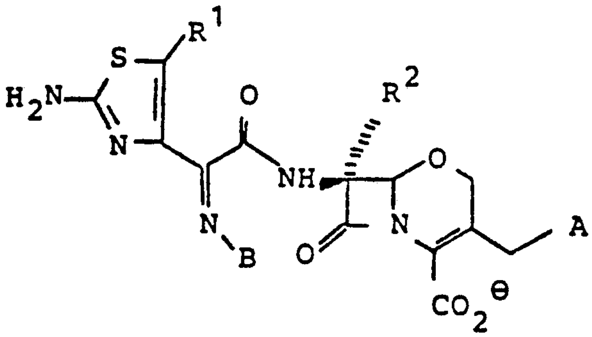

(1)

in welcher
$R^1$ Wasserstoff, Fluor, Chlor oder Brom,
$R^2$ Wasserstoff oder Methoxy bedeutet,
B eine Alkoxygruppe O-$R^3$ bedeutet, in der
$R^3$ für $C_1$-$C_6$-Alkyl oder für einen Rest -$(CH_2)_n(Z)_mR^4$ steht, in dem
$R^4$ eine Gruppe $CO_2R^5$ darstellt, in der
$R^5$ Wasserstoff, $C_1$-$C_4$-Alkyl, $CH_2OC_1$-$C_4$-Alkyl oder ein Äquivalent eines Alkalimetalls, Erdalkalimetalls, Ammonium oder einer organischen Aminbase bedeutet, oder
eine Nitrilgruppe oder Carbamoylgruppe bedeutet, und
n, m jeweils 0 oder 1 bedeutet oder
B für

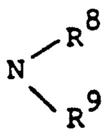, 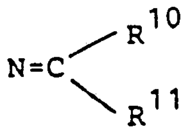, 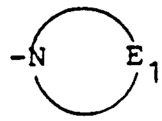 oder 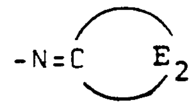 steht,

wobei

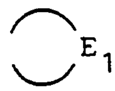

$-(CO)-(NR^{12})-(CH_2)_{n'}-$, $-(CS)-(NR^{12})-(CH_2)_{n'}-$
$-(CH_2)_{m'}-R^{13}-(CH_2)_{m'}-$
$-(CO)_{m'}-(CH_2)_p-$
$-(CO)_{2-m'}-(CH_2)_{n'}-(CO)_{m'}$
$-CO-CH=CH-CO-$
$-CO-O-(CH_2)_q-$
$-CO-S-(CH_2)_q-$
$-CO-NR^{12}-CO(CH_2)_{m'}-$
$-SO_2-(CH_2)_p-$
$-CO-(CH_2)_q-SO_2-$
$-CO-NR^{12}-N=CH-$
$-(CH=CH)_2-$
$-CO-NH-N=CH-CO-$
oder
$CO-(CH=CH)_2$
bedeutet, wobei
$R^{12}$ entweder die gleiche Bedeutung haben kann wie $R^8$ oder

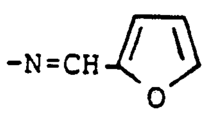

oder $-NHR^8$ bedeutet,
$R^{13}$ für O, S, SO, $SO_2$ oder $NR^{14}$ steht, wobei
$R^{14}$ Wasserstoff, Methyl, Ethyl, Cyclopropyl, Methylsulfonyl oder Furylidenamino bedeutet und
m' 1 oder 2,
n' 2 oder 3,
p 3, 4 oder 5 und
q 2, 3 oder 4 ist,
$E_2$ für einen organischen Rest, der mit dem Kohlenstoffatom einen 4- bis 7-gliedrigen carbocyclischen oder heterocyclischen Ring bildet, der 1 oder 2 Sauerstoff-, Schwefel- oder Stickstoffatome enthalten kann,
$R^8$, $R^9$, $R^{10}$ und $R^{11}$ unabhängig voneinander für $C_1$- bis $C_4$-Alkyl, Benzyl, Phenylethyl, Cyclohexyl oder Phenyl oder einen heterocyclischen Rest wie

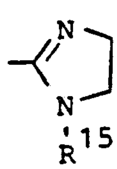, 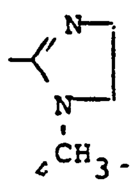, 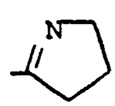, 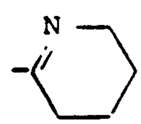,

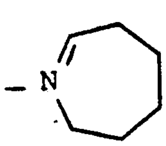, 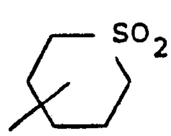, 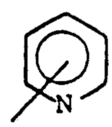, 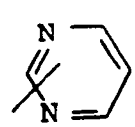,

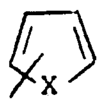, 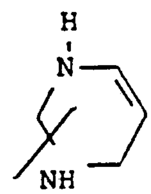, 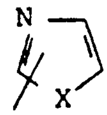, 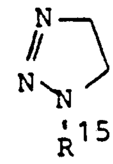,

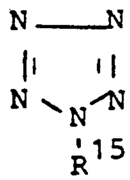, 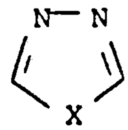,

stehen, wobei

$R^{15}$ für Wasserstoff oder $C_1$-$C_4$-Alkyl, Hydroxyethyl oder $C_1$-$C_4$-Alkoxyethyl und

X für S, O, SO, $SO_2$ steht,

Z eine Gruppe 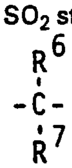

bedeutet, wobei

$R^6$ und $R^7$ gleich oder verschieden sein können und Wasserstoff, eine $C_1$-$C_4$-Alkylgruppe bedeuten, oder zusammen mit dem Kohlenstoff, an das sie gebunden sind, eine Methylen- oder eine $C_3$-$C_7$-Cycloalkylidengruppe bilden,

A einen Pyridiniumrest 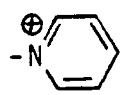

bedeutet, der entweder unsubstituiert ist oder ein- oder mehrfach, gleich oder verschieden substituiert ist,
durch $C_1$-$C_6$-Alkyl,
durch Cyano-$C_1$-$C_3$-alkyl, Epoxy-$C_2$-$C_6$-alkyl, Trifluormethyl oder Pentafluorethyl,
durch Hydroximino-methyl oder $C_1$-$C_4$-Alkoximinomethyl,
durch $C_2$-$C_6$-Alkenyl,
durch $C_2$-$C_6$-Alkinyl,
durch $C_3$-$C_7$-Cycloalkyl oder $C_3$-$C_7$-Cycloalkyl-methyl,
durch $C_4$-$C_7$-Cycloalkenyl,
durch $C_1$-$C_6$-Alkoxy,
durch Epoxy-$C_2$-$C_6$-alkoxy,
durch $C_2$-$C_6$-Alkenyloxy oder $C_2$-$C_6$-Alkinyloxy,
durch Phenoxy oder Heteroaryloxy,
durch Amino,
durch Cyano, Hydroxy oder Mercapto,
durch $C_1$-$C_6$-Alkylthio, $C_1$-$C_6$-Alkylsulfinyl oder $C_1$-$C_6$-Alkylsulfonyl,
durch $C_2$-$C_6$-Alkenylthio, $C_2$-$C_6$-Alkenylsulfinyl oder $C_2$-$C_6$-Alkenylsulfonyl,
durch Phenyl, Benzyl oder Heteroaryl,
durch Formyl,
durch $C_1$-$C_6$-Alkylcarbonyl,
durch Benzoyl,
durch $C_1$-$C_6$-Alkylcarbonylamino,
durch Carboxy oder $C_1$-$C_6$-Alkoxycarbonyl,
durch Sulfamoyl und an den ein oder zwei 3- bis 7-gliedrige Ringe ankondensiert sein können, die jeweils bis zu zwei Doppelbindungen enthalten können und auch aromatisch oder heteroaromatisch sein können.

2. 1-Oxadethiacephalosporinderivate nach Anspruch 1, dadurch gekennzeichnet, daß $R^1$, $R^2$ und B die in Anspruch 1 angegebene Bedeutung haben und

A einen Pyridiniumrest 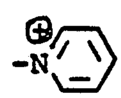

bedeutet, der entweder unsubstituiert ist, oder ein- oder mehrfach, gleich oder verschieden substituiert ist durch $C_1$-$C_6$-Alkyl, das ein- oder mehrfach substituiert ist, durch Hydroxy, Carboxy, $C_1$-$C_6$-Alkyloxycarbonyl, Formyl oder $C_1$-$C_6$-Alkylcarbonyl, Carbamoyl, N-Hydroxycarbamoyl, Sulfo, $C_1$-$C_6$-Alkyloxy, Hydroxy-$C_1$-$C_6$-Alkyloxy, $C_1$-$C_6$-Alkylthio, $C_1$-$C_6$-Alkylsulfinyl, $C_1$-$C_6$-Alkylsulfonyl, $C_2$-$C_6$-Alkenyloxy, $C_2$-$C_6$-Alkenylthio, $C_2$-$C_6$-Alkenylsulfinyl oder $C_2$-$C_6$-Alkenylsulfonyl,

durch Cyano-$C_1$-$C_3$-alkyl, Epoxy-$C_2$-$C_6$-alkyl, Hydroxyiminomethyl, $C_1$-$C_4$-Alkoxyiminomethyl, Trifluormethyl oder Pentafluorethyl,

durch $C_2$-$C_6$-Alkenyl, das durch Hydroxy substituiert sein kann, durch $C_2$-$C_6$-Alkinyl, durch $C_3$-$C_7$-Cycloalkyl oder $C_3$-$C_7$-Cycloalkylmethyl, wobei in beiden Substituenten der Ring auch substituiert sein kann durch Hydroxy, Halogen, Carboxy, $C_1$-$C_6$-Alkyloxycarbonyl oder Cyano,

durch $C_4$-$C_7$-Cycloalkenyl,

durch $C_1$-$C_8$-Alkoxy, das durch Hydroxy, Carboxy oder $C_1$-$C_6$-Alkyloxycarbonyl substituiert sein kann,

durch Epoxy-$C_2$-$C_6$-alkoxy,

durch $C_2$-$C_6$-Alkenyloxy oder $C_2$-$C_6$-Alkinyloxy,

durch Phenoxy,

durch Amino, das ein- oder zweifach, gleich oder verschieden substituiert sein kann durch $C_1$-$C_6$-Alkyl, Formyl, $C_1$-$C_6$-Alkylcarbonyl, $C_1$-$C_6$-Alkoxycarbonyl, Carbamoyl, $C_1$-$C_6$-Alkylsulfonyl,

durch Cyano, Hydroxy oder Mercapto,

durch $C_1$-$C_6$-Alkylthio, $C_1$-$C_6$-Alkylsulfinyl oder $C_1$-$C_6$-Alkylsulfonyl, die im Alkylteil durch Hydroxy substituiert sein können,

durch Methylthio, Methylsulfinyl oder Methylsulfonyl, die im Methylteil substituiert sind durch Carboxy oder $C_1$-$C_6$-Alkyloxycarbonyl,

durch $C_2$-$C_6$-Alkenylthio, $C_2$-$C_6$-Alkenylsulfinyl oder $C_2$-$C_6$-Alkenylsulfonyl,

durch Phenyl oder Benzyl, die auch durch Halogen substituiert sein können,

durch Formyl,

durch $C_1$-$C_6$-Alkylcarbonyl, das auch durch Hydroxy substituiert sein kann,

durch Benzoyl oder $C_1$-$C_6$-Alkylcarbonylamino,

durch Carboxy oder $C_1$-$C_6$-Alkoxycarbonyl,

durch Sulfamoyl, das am Stickstoff einmal substituiert sein kann durch $C_1$-$C_6$-Alkylaminocarbonyl, und an den ein 3- bis 7-gliedriger Ring ankondensiert sein kann, der bis zu zwei Heteroatome aus der Gruppe O, N und S, und bis zu zwei Doppelbindungen enthalten kann und auch aromatisch oder heteroaromatisch sein kann und der durch $C_1$-$C_6$-Alkyl, $C_3$-$C_7$-Cycloalkyl, $C_1$-$C_4$-Alkoxy, $C_1$-$C_4$-Hydroxyalkyl, Halogen, Hydroxy, Oxo, Hydroximino, Exomethylen, Carboxy, $C_1$-$C_6$-Alkyloxycarbonyl, Cyano, Carbamoyl, Sulfamoyl, Amino, $C_1$-$C_4$-Alkylamino oder $C_1$-$C_4$-Dialkylamino ein- oder mehrfach substituiert sein kann.

3. 1-Oxadethiacephalosporinderivate nach Anspruch 1 aus der Gruppe bestehend aus

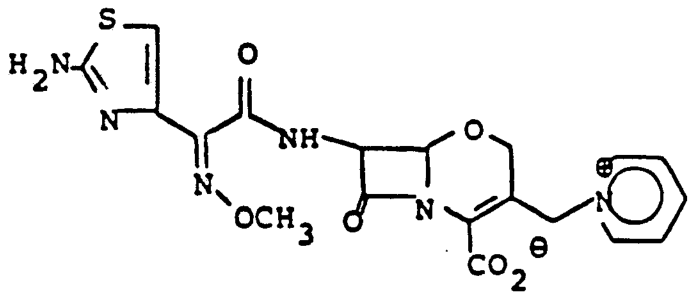

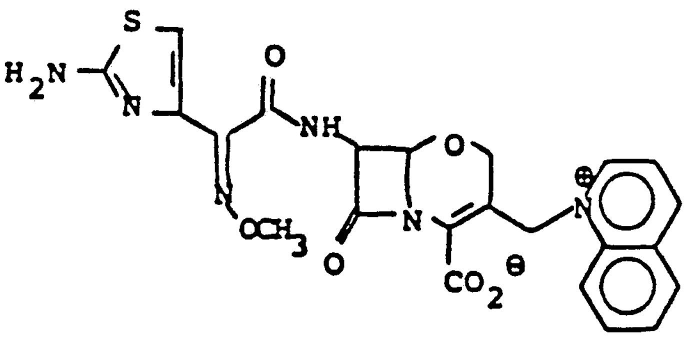

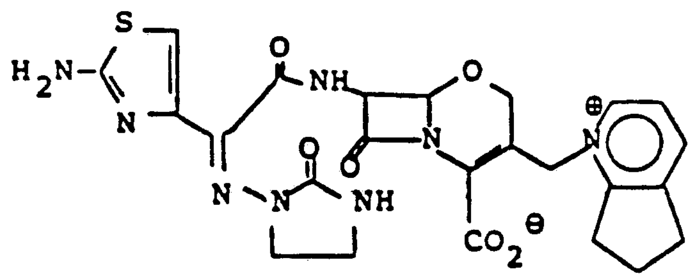

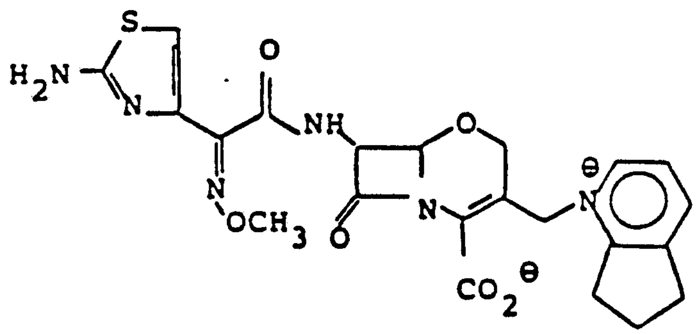

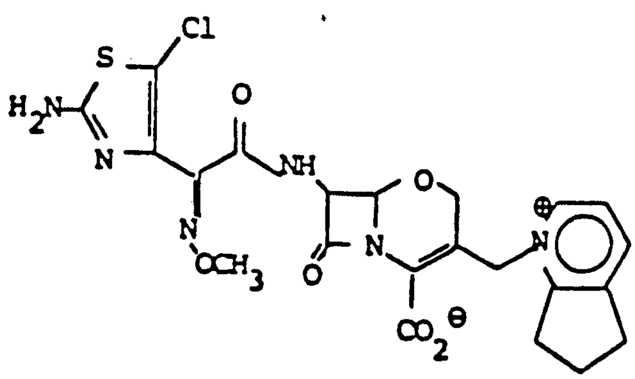

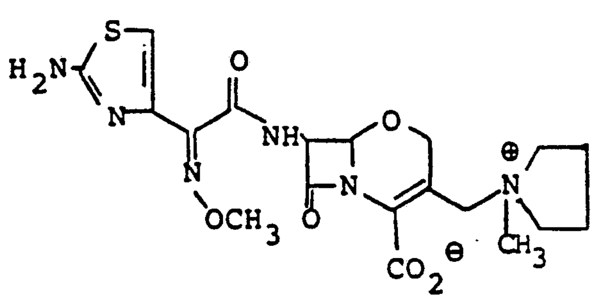

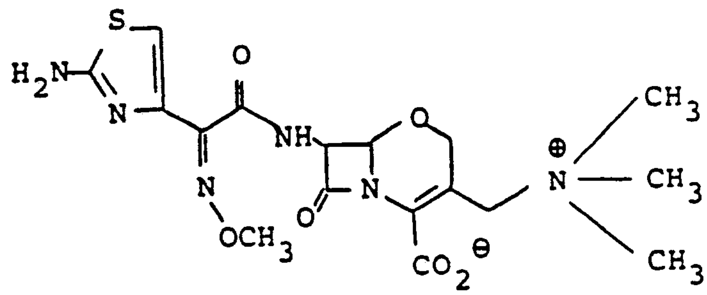

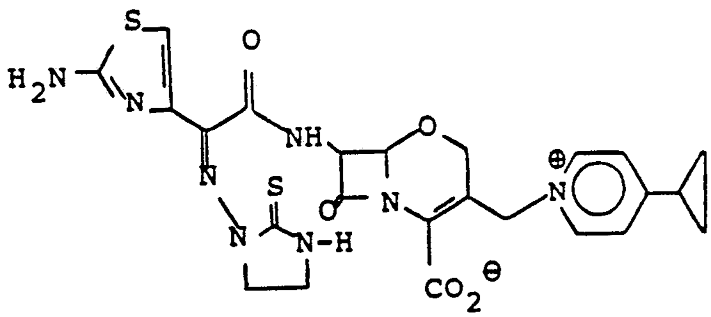

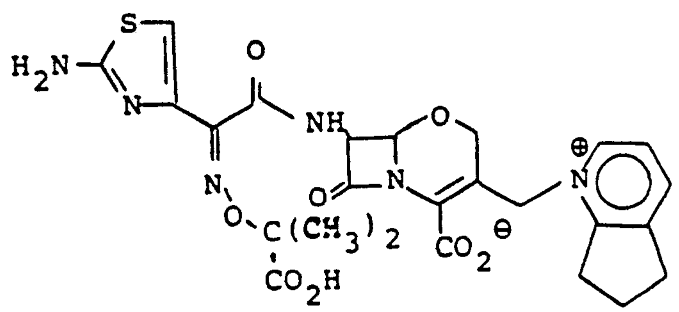

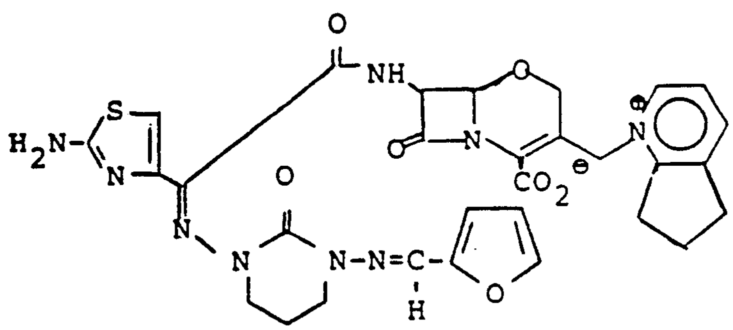

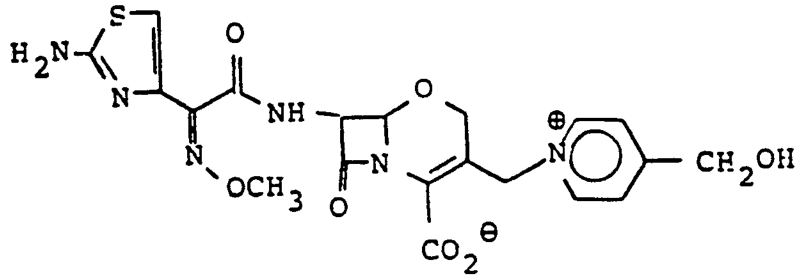

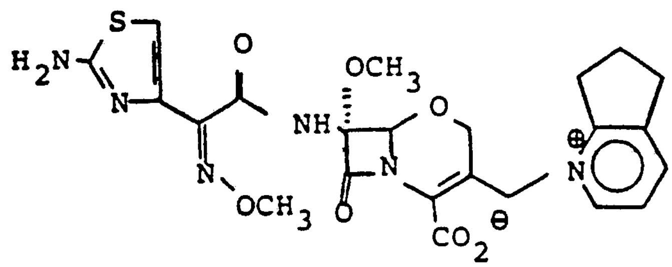

4. Verfahren zur Herstellung von 1-Oxadethiacephalosporinderivaten der allgemeinen Formel 1,

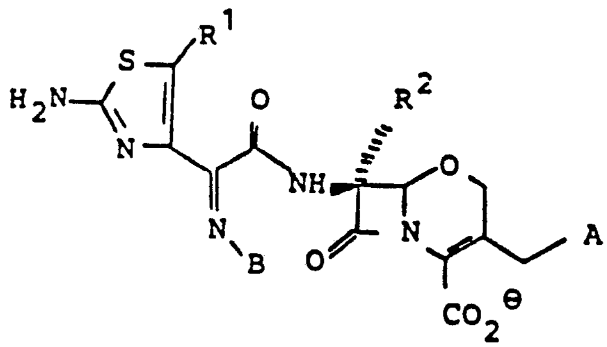

(1)

gemäß Anspruch 1, dadurch gekennzeichnet, daß man

a) Verbindungen der allgemeinen Formel 2,

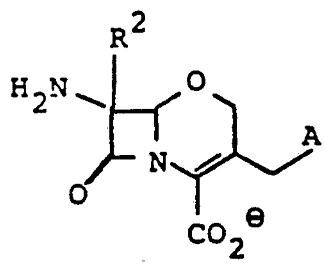

(2)

in der

A bzw. $R^2$ die in Anspruch 1 angegebene Bedeutung haben, wobei die Aminogruppe auch in der Form eines reaktionsfähigen Derivats vorliegen kann,

mit einer 2-(2-Aminothiazol-4-yl)-2-syn-oximinoessigsäure der allgemeinen Formel 3,

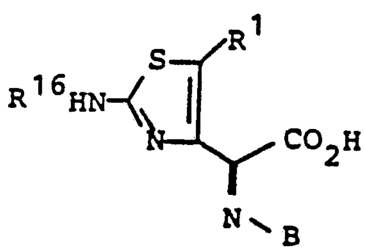

(3)

in der

B und $R^1$ die in Anspruch 1 angegebene Bedeutung haben und

$R^{16}$ Wasserstoff oder eine Aminoschutzgruppe darstellt,

in Form eines aktivierten Derivates dieser Verbindung umsetzt und eine gegebenenfalls vorhandene Schutzgruppe abspaltet, oder

b) eine Verbindung der allgemeinen Formel 4,

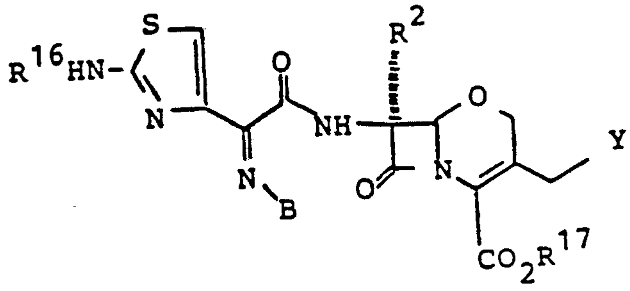

(4)

in der
R¹, R², R¹⁶ und B die in Anspruch 1 angegebene Bedeutung haben und
$R^{17}$ Wasserstoff oder einen organischen Ammoniumrest,
Y eine nucleofuge Abgangsgruppe vorzugsweise Chlor, Brom oder Jod, Chloracetoxy, Dichloracetoxy, Mesyloxy oder Tosyloxy bedeuten, mit Pyridin oder einem Pyridinderivat, das einem in Formel 1 genannten Pyridiniumrest A entspricht, umsetzt, worauf man, falls der Rest $R^{16}$ eine leicht abspaltbare Schutzgruppe darstellt, diese hydrolytisch oder hydrogenolytisch abgespaltet und so die Verbindungen der allgemeinen Formel 1 erhält.

5. Arzneimittel enthaltend mindestens eine Verbindung nach einem der Ansprüche 1 - 3.

6. Verbindungen gemäß Formel 1 in den Ansprüchen 1 - 3 zur Verwendung bei der therapeutischen Behandlung des menschlichen oder tierischen Körpers.

7. Verwendung von Verbindungen nach den Ansprüchen 1 - 3 zur Herstellung von Arzneimitteln.

**Patentansprüche** für der Vertragsstaat: Österreich

1. Verfahren zur Herstellung von 1-Oxadethiacephalosporinderivaten der allgemeinen Formel 1

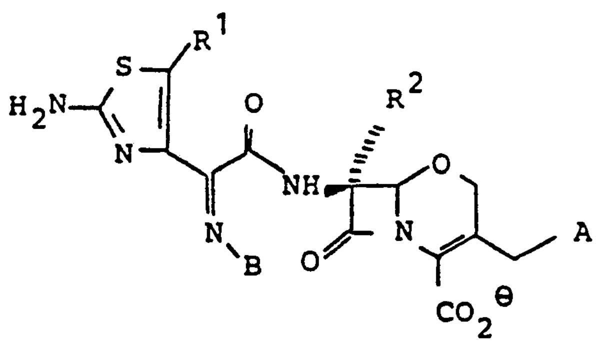

(1)

in welcher
$R^1$ Wasserstoff, Fluor, Chlor oder Brom,
$R^2$ Wasserstoff oder Methoxy bedeutet,
B eine Alkoxygruppe O-$R^3$ bedeutet, in der
$R^3$ für $C_1$-$C_6$-Alkyl oder für einen Rest $-(CH_2)_n(Z)_mR^4$ steht, in dem
$R^4$ eine Gruppe $CO_2R^5$ darstellt, in der
$R^5$ Wasserstoff, $C_1$-$C_4$-Alkyl, $CH_2OC_1$-$C_4$-Alkyl oder ein Äquivalent eines Alkalimetalls, Erdalkalimetalls, Ammonium oder einer organischen Aminbase bedeutet, oder
eine Nitrilgruppe oder Carbamoylgruppe bedeutet, und
n, m jeweils 0 oder 1 bedeutet oder
B für

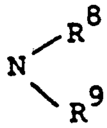 , 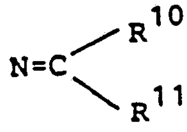 , 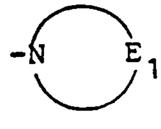 oder 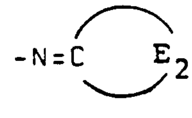 steht,

wobei

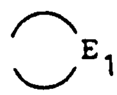

$-(CO)-(NR^{12})-(CH_2)_{n'}-$, $-(CS)-(NR^{12})-(CH_2)_{n'}-$
$-(CH_2)_{m'}-R^{13}-(CH_2)_{m'}-$
$-(CO)_{m'}-(CH_2)_p-$
$-(CO)_{2-m'}-(CH_2)_{n'}-(CO)_{m'}$
$-CO-CH=CH-CO-$
$-CO-O-(CH_2)_q-$
$-CO-S-(CH_2)_q-$
$-CO-NR^{12}-CO(CH_2)_{m'}-$
$-SO_2-(CH_2)_p-$
$-CO-(CH_2)_q-SO_2-$
$-CO-NR^{12}-N=CH-$
$-(CH=CH)_2-$
$-CO-NH-N=CH-CO-$
oder
$CO-(CH=CH)_2$
bedeutet, wobei
$R^{12}$ entweder die gleiche Bedeutung haben kann wie $R^8$ oder

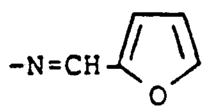

oder $-NHR^8$ bedeutet,
$R^{13}$ für O, S, SO, $SO_2$ oder $NR^{14}$ steht, wobei
$R^{14}$ Wasserstoff, Methyl, Ethyl, Cyclopropyl, Methylsulfonyl oder Furylidenamino bedeutet und
m' 1 oder 2,
n' 2 oder 3,
p 3, 4 oder 5 und
q 2, 3 oder 4 ist,

$E_2$ für einen organischen Rest, der mit dem Kohlenstoffatom einen 4- bis 7-gliedrigen carbocyclischen oder heterocyclischen Ring bildet, der 1 oder 2 Sauerstoff-, Schwefel- oder Stickstoffatome enthalten kann,

$R^8$, $R^9$, $R^{10}$ und $R^{11}$ unabhängig voneinander für $C_1$- bis $C_4$-Alkyl, Benzyl, Phenylethyl, Cyclohexyl oder Phenyl oder einen heterocyclischen Rest wie

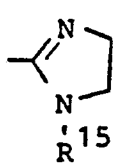 , 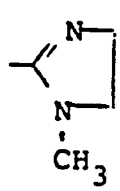 , 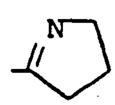 , 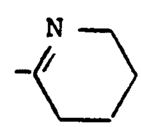 ,

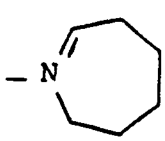 , 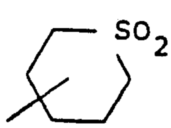 , 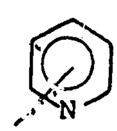 , 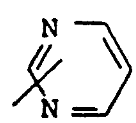 ,

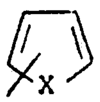 , 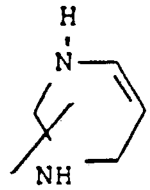 , 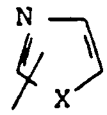 , 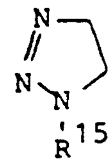 ,

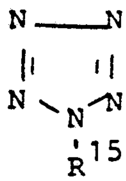 , 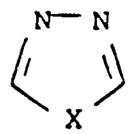 ,

stehen, wobei

$R^{15}$ für Wasserstoff oder $C_1$-$C_4$-Alkyl, Hydroxyethyl oder $C_1$-$C_4$-Alkoxyethyl und

X für S, O, SO, $SO_2$ steht,

Z eine Gruppe 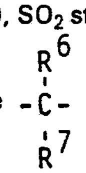

bedeutet, wobei

$R^6$ und $R^7$ gleich oder verschieden sein können und Wasserstoff, eine $C_1$-$C_4$-Alkylgruppe bedeuten, oder zusammen mit dem Kohlenstoff, an das sie gebunden sind, eine Methylen- oder eine $C_3$-$C_7$-Cycloalkylidengruppe bilden,

A einen Pyridiniumrest 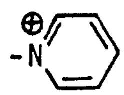

bedeutet, der entweder unsubstituiert ist oder ein- oder mehrfach, gleich oder verschieden substituiert ist,

durch $C_1$-$C_6$-Alkyl,

durch Cyano-$C_1$-$C_3$-alkyl, Epoxy-$C_2$-$C_6$-alkyl, Trifluormethyl oder Pentafluorethyl,

durch Hydroximino-methyl oder $C_1$-$C_4$-Alkoximinomethyl,

durch $C_2$-$C_6$-Alkenyl,

durch $C_2$-$C_6$-Alkinyl,

durch $C_3$-$C_7$-Cycloalkyl oder $C_3$-$C_7$-Cycloalkyl-methyl,

durch $C_4$-$C_7$-Cycloalkenyl,

durch $C_1$-$C_6$-Alkoxy,

durch Epoxy-$C_2$-$C_6$-alkoxy,

durch $C_2$-$C_6$-Alkenyloxy oder $C_2$-$C_6$-Alkinyloxy,

durch Phenoxy oder Heteroaryloxy,

durch Amino,

durch Cyano, Hydroxy oder Mercapto,

durch $C_1$-$C_6$-Alkylthio, $C_1$-$C_6$-Alkylsulfinyl oder $C_1$-$C_6$-Alkylsulfonyl,

durch $C_2$-$C_6$-Alkenylthio, $C_2$-$C_6$-Alkenylsulfinyl oder $C_2$-$C_6$-Alkenylsulfonyl,

durch Phenyl, Benzyl oder Heteroaryl,

durch Formyl,

durch $C_1$-$C_6$-Alkylcarbonyl,

durch Benzoyl,

durch $C_1$-$C_6$-Alkylcarbonylamino,

durch Carboxy oder $C_1$-$C_6$-Alkoxycarbonyl,

durch Sulfamoyl und an den ein oder zwei 3- bis 7-gliedrige Ringe kondensiert sein können, die jeweils bis zu zwei Doppelbindungen enthalten können und auch aromatisch oder heteroaromatisch sein können,

dadurch gekennzeichnet, daß man

a) Verbindungen der allgemeinen Formel 2,

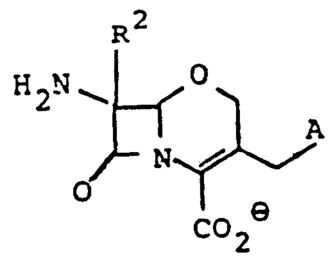

(2)

in der

A bzw. $R^2$ die oben angegebene Bedeutung haben, wobei die Aminogruppe auch in der Form eines reaktionsfähigen Derivats vorliegen kann,

mit einer 2-(2-Aminothiazol-4-yl)-2-syn-oximinoessigsäure der allgemeinen Formel 3,

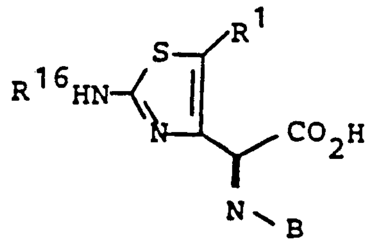

(3)

in der

B und $R^1$ die oben angegebene Bedeutung haben und

$R^{16}$ Wasserstoff oder eine Aminoschutzgruppe darstellt,

in Form eines aktivierten Derivates dieser Verbindung umsetzt und eine gegebenenfalls vorhandene Schutzgruppe abspaltet, oder

b) eine Verbindung der allgemeinen Formel 4,

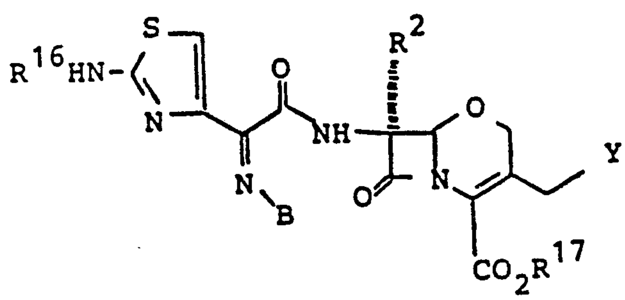

(4)

in der

$R^1$, $R^2$, $R^{16}$ und B die oben angegebene Bedeutung haben und

$R^{17}$ Wasserstoff oder einen organischen Ammoniumrest,

Y eine nucleofuge Abgangsgruppe vorzugsweise Chlor, Brom oder Jod, Chloracetoxy, Dichloracetoxy, Mesyloxy oder Tosyloxy bedeuten,

mit Pyridin oder einem Pyridinderivat, das einem in Formel 1 genannten Pyridiniumrest A entspricht, umsetzt, worauf man, falls der Rest $R^{16}$ eine leicht abspaltbare Schutzgruppe darstellt, diese hydrolytisch oder hydrogenolytisch abgespaltet und so die Verbindungen der allgemeinen Formel 1 erhält.

2. Verfahren nach Anspruch 1, dadurch gekennzeichnet, daß man als Aminoschutzgruppe für $R^{16}$ eine tert. Butoxycarbonyl-, Carbobenzoxy-, Trityl-, Formyl- oder Chloracetylgruppe einsetzt.

3. Verfahren nach Anspruch 1, dadurch gekennzeichnet, daß man die Umsetzung der Verbindungen der Formel 2 mit Carbonsäuren der Formel 3 bzw. mit deren aktivierten Derivaten in einem Temperaturbereich von -80° C bis +80° C durchfuhrt.

4. Verfahren nach Anspruch 3, dadurch gekennzeichnet, daß man die Umsetzung in Gegenwart eines inerten Lösungsmittels durchführt.

5. Verfahren nach Anspruch 4, dadurch gekennzeichnet, daß man die Umsetzung in Dimethylformamid durchführt.

**Claims** for contracting states: BE, DE, FR, IT, NL, CH, SE, GB, LI

1. 1-Oxadethiacephalosporin derivatives of the general formula 1

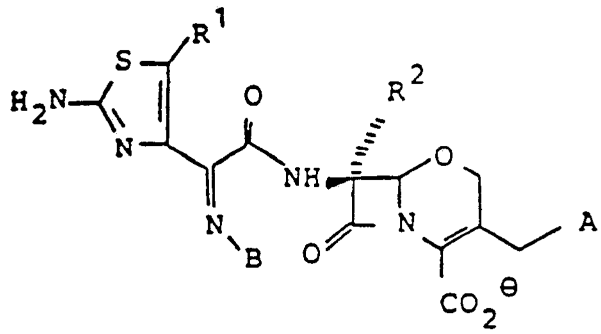

(1)

in which

$R^1$ denotes hydrogen, fluorine, chlorine or bromine,
$R^2$ denotes hydrogen or methoxy,
B denotes an alkoxy group O-$R^3$,
in which
$R^3$ represents $C_1$-$C_6$-alkyl or a $-(CH_2)_n(Z)_mR^4$ radical,
in which
$R^4$ represents a $CO_2R^5$ group
in which
$R^5$ denotes hydrogen, $C_1$-$C_4$-alkyl, $CH_2OC_1$-$C_4$-alkyl or one equivalent of an alkali metal, alkaline earth metal, ammonium or an organic amine base, or denotes a nitrile group or carbamoyl group, and
n and m each denote 0 or 1, or B represents

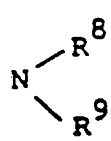 , 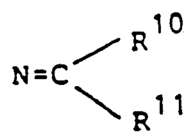 , 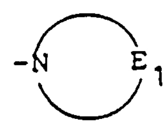 or 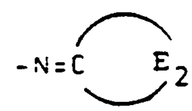

wherein

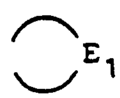

denotes
$-(CO)-(NR^{12})-(CH_2)_{n''}-$, $-(CS)-(NR^{12})-(CH_2)_{n''}-$
$-(CH_2)_{m''}-R^{13}-(CH_2)_{m''}-$
$-(CO)_{m''}-(CH_2)_p-$
$-(CO)_{2-m''}-(CH_2)_{n''}-(CO)_{m'}$
$-CO-CH=CH-CO-$
$-CO-O-(CH_2)_q-$
$-CO-S-(CH_2)_q-$
$-CO-NR^{12}-CO(CH_2)_{m''}-$
$-SO_2-(CH_2)_p-$
$-CO-(CH_2)_q-SO_2-$
$-CO-NR^{12}-N=CH-$
$-(CH=CH)_2-$
$-CO-NH-N=CH-CO-$
or
$CO-(CH=CH)_2$ wherein
$R^{12}$ can either have the same meaning as $R^8$ or denotes

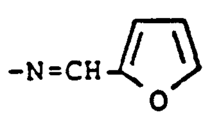

or $-NHR^8$,

$R^{13}$ represents O, S, SO, $SO_2$ or $NR^{14}$, wherein
$R^{14}$ denotes hydrogen, methyl, ethyl, cyclopropyl, methylsulphonyl or furylideneamino and
m' is 1 or 2,
n' is 2 or 3,
p is 3, 4 or 5 and
q is 2, 3 or 4,
$E_2$ represents an organic radical which, with the carbon atom, forms a 4-membered to 7-membered carbocyclic or heterocyclic ring which can contain 1 or 2 oxygen, sulphur or nitrogen atoms,
$R^8$, $R^9$, $R^{10}$ and $R^{11}$ independently of one another represent $C_1$ to $C_4$-alkyl, benzyl, phenylethyl, cyclohexyl or phenyl, or a heterocyclic radical such as

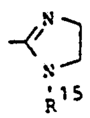 , 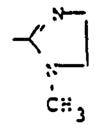 , 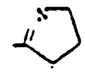 , 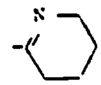 ,

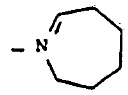 , 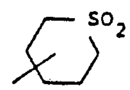 , 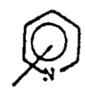 , 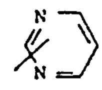 ,

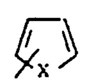 , 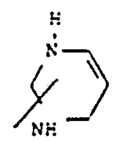 , 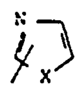 , 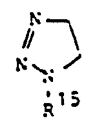 ,

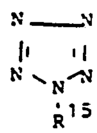 , 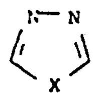 ,

wherein
$R^{15}$ represents hydrogen or $C_1$-$C_4$-alkyl, hydroxyethyl or $C_1$-$C_4$-alkoxyethyl, and
X represents S, O, SO or $SO_2$,

Z denotes a 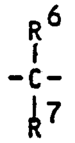 group, wherein

$R^6$ and $R^7$ can be identical or different and denote hydrogen or a $C_1$-$C_4$-alkyl group, or, together with the carbon to which they are bonded form a methylene or a $C_3$-$C_7$-cycloalkylidene group,

A denotes a pyridinium radical −N which is either unsubstituted or mono- or polysubstituted, identically or differently, by $C_1$-$C_6$-alkyl, by cyano-$C_1$-$C_3$-alkyl, epoxy-$C_2$-$C_6$-alkyl, trifluoromethyl or pentafluoroethyl, by hydroximinomethyl or $C_1$-$C_4$-alkoximinomethyl, by $C_2$-$C_6$-alkenyl, by $C_2$-$C_6$-alkinyl, by $C_3$-$C_7$-cycloalkyl or $C_3$-$C_7$-cycloalkylmethyl, by $C_4$-$C_7$-cycloalkenyl, by $C_1$-$C_6$-alkoxy, by epoxy-$C_2$-$C_6$-alkoxy, by $C_2$-$C_6$-alkenyloxy or $C_2$-$C_6$-alkinyloxy, by phenoxy or heteroaryloxy, by amino, by cyano, hydroxyl or mercapto, by $C_1$-$C_6$-alkylthio, $C_1$-$C_6$-alkylsulphinyl or $C_1$-$C_6$-alkylsulphonyl, by $C_2$-$C_6$-alkenylthio, $C_2$-$C_6$-alkenylsulphinyl or $C_2$-$C_6$-alkenylsulphonyl, by phenyl, benzyl or heteroaryl, by formyl, by $C_1$-$C_6$-alkylcarbonyl, by benzoyl, by $C_1$-$C_6$-alkylcarbonylamino, by carboxyl or $C_1$-$C_6$-alkoxycarbonyl, or by sulphamoyl, and to which one or two 3-membered to 7-membered rings can be fused which can each contain up to two double bonds and can also be aromatic or heteroaromatic.

2. 1-Oxadethiacephalosporin derivatives according to Claim 1, characterized in that
$R^1$, $R^2$ and B have the meaning given in Claim 1 and

A denotes a pyridinium radical 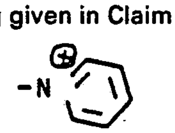

which is either unsubstituted or mono- or polysubstituted, identically or differently, by $C_1$-$C_6$-alkyl which is mono- or polysubstituted, by hydroxyl, carboxyl, $C_1$-$C_6$-alkoxycarbonyl, formyl or $C_1$-$C_6$-alkylcarbonyl, carbamoyl, N-hydroxycarbamoyl, sulpho, $C_1$-$C_6$-alkyloxy, hydroxy-$C_1$-$C_6$-alkoxy, $C_1$-$C_6$-alkylthio, $C_1$-$C_6$-alkylsulphinyl, $C_1$-$C_6$-alkylsulphonyl, $C_2$-$C_6$-alkenyloxy, $C_2$-$C_6$-alkenylthio, $C_2$-$C_6$-alkenylsulphinyl or $C_2$-$C_6$-alkenylsulphonyl, by cyano-$C_1$-$C_3$-alkyl, epoxy-$C_2$-$C_6$-alkyl, hydroxyiminomethyl, $C_1$-$C_4$-alkoxyiminomethyl,

trifluoromethyl or pentafluoroethyl, by $C_2$-$C_6$-alkenyl which can be substituted by hydroxyl, by $C_2$-$C_6$-alkinyl, by $C_3$-$C_7$-cycloalkyl or $C_3$-$C_7$-cycloalkylmethyl,

wherein

in both substitutents the ring can also be substituted by hydroxyl, halogen, carboxyl, $C_1$-$C_6$-alkoxycarbonyl or cyano, by $C_4$-$C_7$-cycloalkenyl, by $C_1$-$C_6$-alkoxy which can be substituted by hydroxyl, carboxyl or $C_1$-$C_6$-alkoxycarbonyl, by epoxy-$C_2$-$C_6$-alkoxy, by $C_2$-$C_6$-alkenyloxy or $C_2$-$C_6$-alkinyloxy, by phenoxy, by amino which can be mono- or disubstituted, identically or differently, by $C_1$-$C_6$-alkyl, formyl, $C_1$-$C_6$-alkylcarbonyl, $C_1$-$C_6$-alkoxycarbonyl, carbamoyl, $C_1$-$C_6$-alkylsulphonyl, by cyano, hydroxyl or mercapto, by $C_1$-$C_6$-alkylthio, $C_1$-$C_6$-alkylsulphinyl or $C_1$-$C_6$-alkylsulphonyl which can be substituted in the alkyl part by hydroxyl, by methylthio, methylsulphinyl or methylsulphonyl which are substituted in the methyl part by carboxyl or $C_1$-$C_6$-alkoxycarbonyl, by $C_2$-$C_6$-alkenylthio, $C_2$-$C_6$-alkenylsulphinyl or $C_2$-$C_6$-alkenylsulphonyl, by phenyl or benzyl which can also be substituted by halogen, by formyl, by $C_1$-$C_6$-alkylcarbonyl which can also be substituted by hydroxyl, by benzoyl or $C_1$-$C_6$-alkylcarbonylamino, by carboxyl or $C_1$-$C_6$-alkoxycarbonyl, by sulfamoyl which can be monosubstituted on the nitrogen by $C_1$-$C_6$-alkylaminocarbonyl and to which a 3-membered to 7-membered ring can be fused which can contain up to two hereto atoms from the group comprising O, N and S and up to two double bonds, and can also be aromatic or heteroaromatic and which can be mono- or polysubstituted by $C_1$-$C_6$-alkyl, $C_3$-$C_7$-cycloalkyl, $C_1$-$C_4$-alkoxy, $C_1$-$C_4$-hydroxyalkyl, halogen, hydroxyl, oxo, hydroximino, exomethylene, carboxyl, $C_1$-$C_6$-alkoxycarbonyl, cyano, carbamoyl, sulphamoyl, amino, $C_1$-$C_4$-alkylamino or $C_1$-$C_4$-dialkylamino.

3. 1-Oxadethiacephalosporin derivatives according to Claim 1 from the group comprising

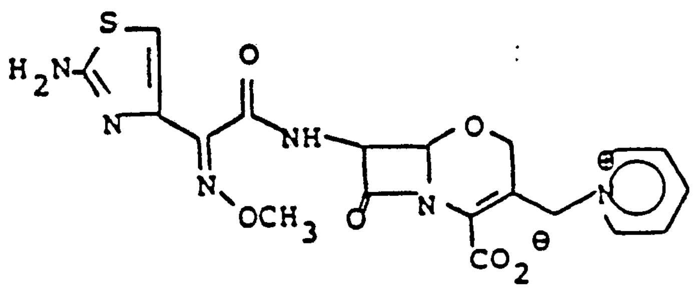

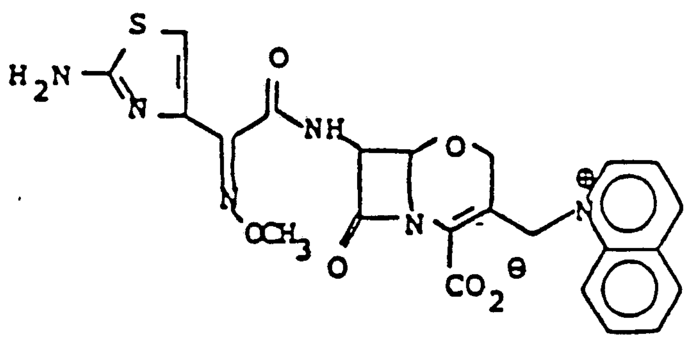

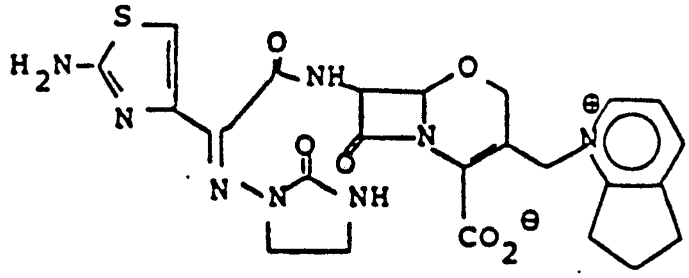

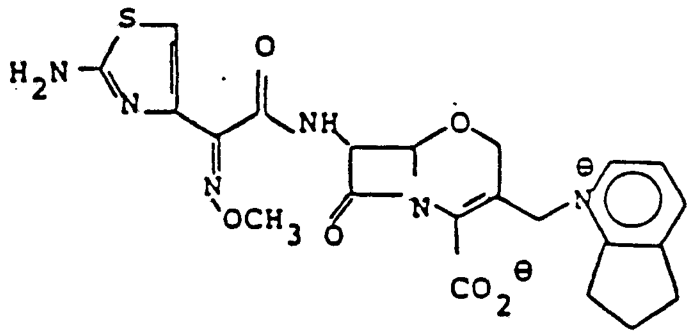

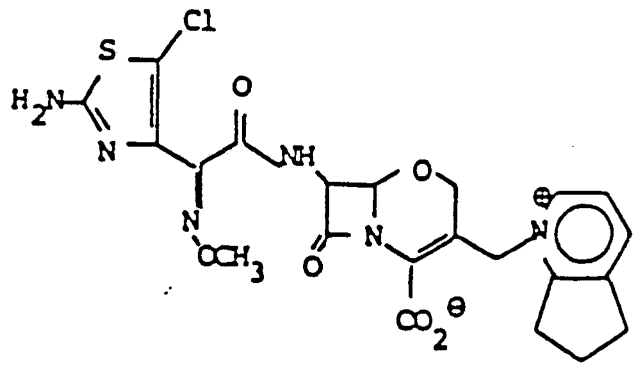

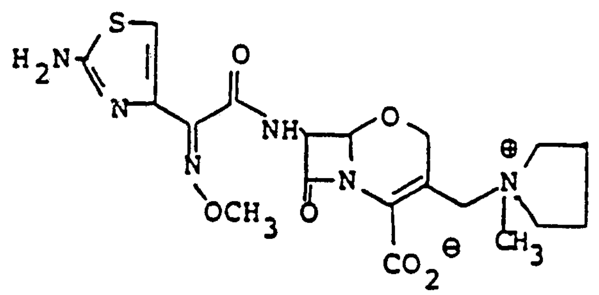

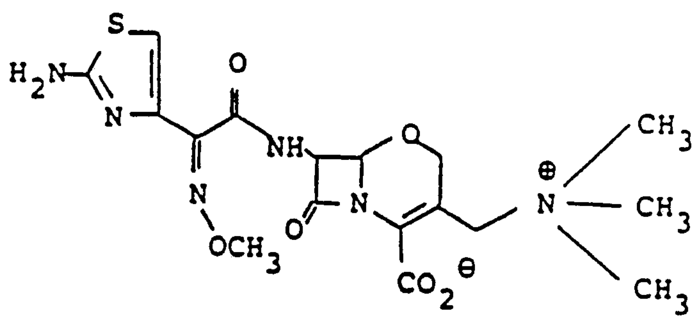

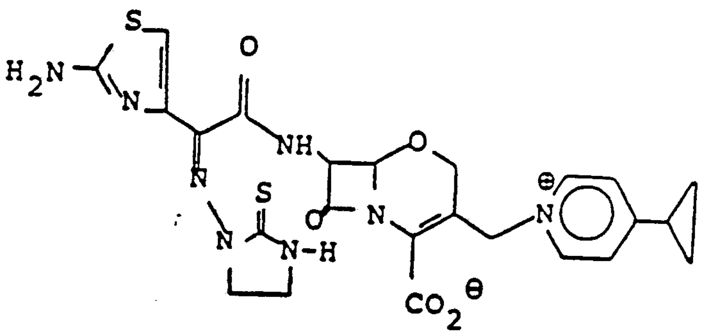

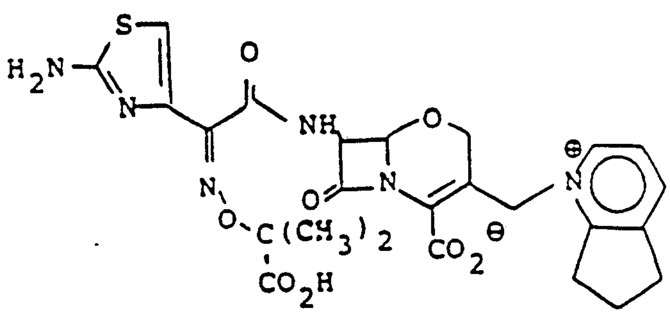

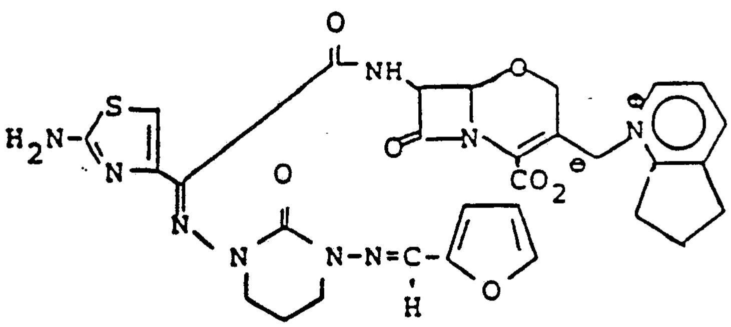

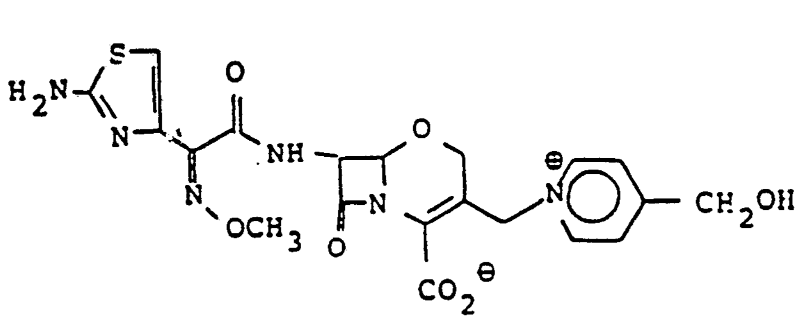

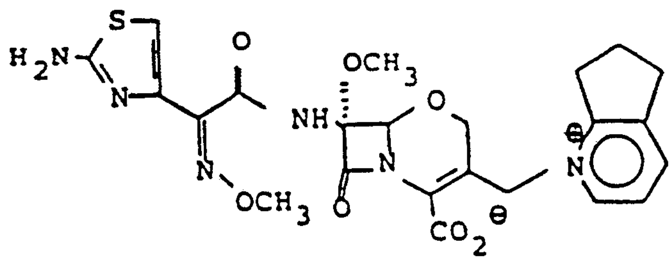

4. Process for the preparation of 1-oxadethiacephalosporin derivatives of the general formula 1 in which

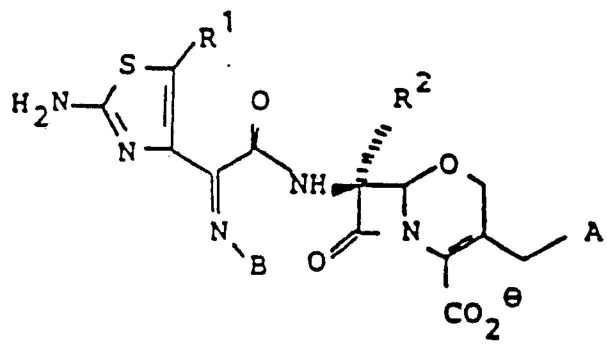

characterized in that

a) compounds of the general formula 2

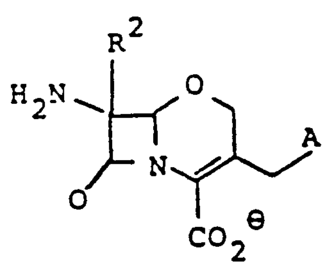

(2)

in which

A and $R^2$ have the meaning given in Claim 1 and the amino group can also be present in the form of a reactive derivative,

are reacted with a 2-(2-aminothiazol-4-yl)-2-syn-oximinoacetic acid of the general formula 3

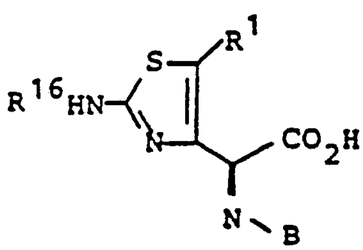

(3)

in which

B and R have the meaning given in Claim 1 and

$R^{16}$ represents hydrogen or an amino-protective group

in the form of an activated derivative of this compound, and a protective group which may be present is split off, or

b) a compound of the general formula 4

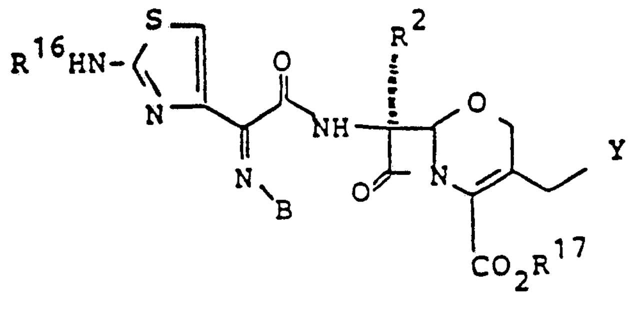

(4)

in which

$R^1$, $R^2$, $R^{16}$ and B have the meaning given in Claim 1, and

$R^{17}$ denotes hydrogen or an organic ammonium radical, and

Y denotes a nucleofugic leaving group, preferably chlorine, bromine or iodine, chloroacetoxy, dichloroacetoxy, mesyloxy or tosyloxy,

is reacted with pyridine or a pyridine derivative which corresponds to a pyridinium radical A mentioned in formula 1, after which, if the radical $R^{16}$ is an easily removable protective group, this group is removed hydrolytically or hydrogenolytically, thus giving the compounds of the general formula 1.

5. Medicaments containing at least one compound according to one of Claims 1 - 3.

6. Compounds according to formula 1 in Claims 1 - 3 for use in the therapeutic treatment of the human or animal body.

7. Use of compounds according to Claims 1 - 3 for the preparation of medicaments.

**Claims** for the contracting state: AT

1. Process for the preparation of 1-oxadethiacephalosporin derivatives of the general formula 1

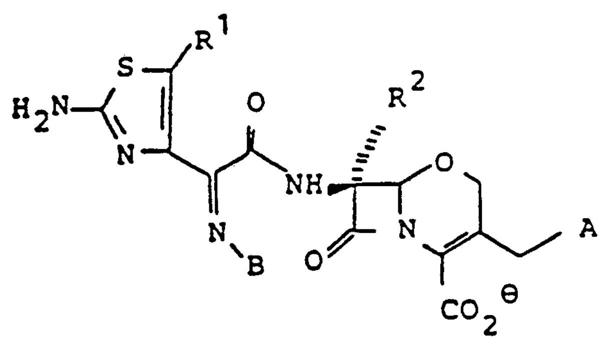

(1)

in which

$R^1$ denotes hydrogen, fluorine, chlorine or bromine,

$R^2$ denotes hydrogen or methoxy,

B denotes an alkoxy group $O-R^3$,

in which

$R^3$ represents $C_1$-$C_6$-alkyl or a $-(CH_2)_n(Z)_mR^4$ radical,

in which

$R^4$ represents a $CO_2R^5$ group

in which

$R^5$ denotes hydrogen, $C_1$-$C_4$-alkyl, $CH_2OC_1$-$C_4$-alkyl or one equivalent of an alkali metal, alkaline earth metal, ammonium or an organic amine base, or denotes a nitrile group or carbamoyl group, and

n and m each denote 0 or 1,

or

B represents

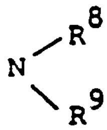, 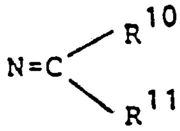, 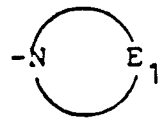 or 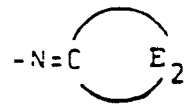

wherein

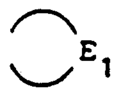

denotes

$-(CO)-(NR^{12})-(CH_2)_{n'}-$, $-(CS)-(NR^{12})-(CH_2)_{n'}-$
$-(CH_2)_{m'}-R^{13}-(CH_2)_{m'}-$
$-(CO)_{m'}-(CH_2)_p-$
$-(CO)_{2-m'}-(CH_2)_{n'}-(CO)_{m'}$
$-CO-CH=CH-CO-$
$-CO-O-(CH_2)_q-$
$-CO-S-(CH_2)_q-$
$-CO-NR^{12}-CO(CH_2)_{m'}-$
$-SO_2-(CH_2)_p-$
$-CO-(CH_2)_q-SO_2-$
$-CO-NR^{12}-N=CH-$
$-(CH=CH)_2-$
$-CO-NH-N=CH-CO-$
or
$CO-(CH=CH)_2$
wherein

$R^{12}$ can either have the same meaning as $R^8$ or denotes 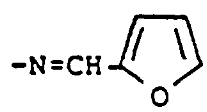

or $-NHR^8$
$R^{13}$ represents O, S, SO, $SO_2$ or $NR^{14}$,
wherein
$R^{14}$ denotes hydrogen, methyl, ethyl, cyclopropyl, methylsulphonyl or furylideneamino and
m' is 1 or 2,
n' is 2 or 3,
p is 3, 4 or 5 and
q is 2, 3 or 4,

$E_2$ represents an organic radical which, with the carbon atom, forms a 4-membered to 7-membered carbocyclic or heterocyclic ring which can contain 1 or 2 oxygen, sulphur or nitrogen atoms,

$R^8$, $R^9$, $R^{10}$ and $R^{11}$ independently of one another represent $C_1$ to $C_4$-alkyl, benzyl, phenylethyl, cyclohexyl or phenyl, or a heterocyclic radical such as

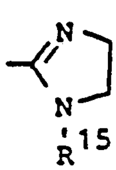, 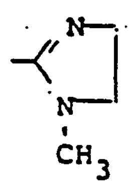, 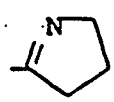, 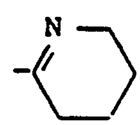,

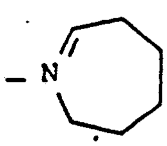, 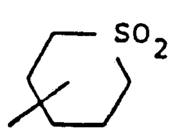, 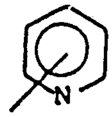, 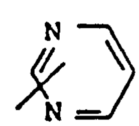,

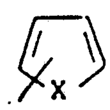 , 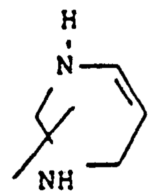 , 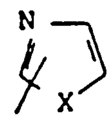 , 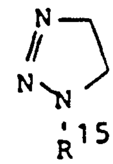 ,

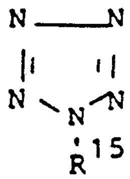 , 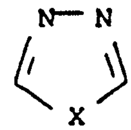 ,

wherein

$R^{15}$ represents hydrogen or $C_1$-$C_4$-alkyl, hydroxyethyl or $C_1$-$C_4$-alkoxyethyl, and

X represents S, O, SO or $SO_2$,

Z denotes a 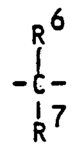 group,

wherein

$R^6$ and $R^7$ can be identical or different and denote hydrogen or a $C_1$-$C_4$-alkyl group, or, together with the carbon to which they are bonded form a methylene or a $C_3$-$C_7$-cycloalkylidene group,

A denotes a pyridinium radical 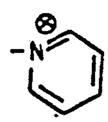

which is either unsubstituted or mono- or polysubstituted, identically or differently, by $C_1$-$C_6$-alkyl, by cyano-$C_1$-$C_3$-alkyl, epoxy-$C_2$-$C_6$-alkyl, trifluoromethyl or pentafluoroethyl, by hydroximinomethyl or $C_1$-$C_4$-alkoximinomethyl, by $C_2$-$C_6$-alkenyl, by $C_2$-$C_6$-alkinyl, by $C_3$-$C_7$-cycloalkyl or $C_3$-$C_7$-cycloalkylmethyl, by $C_4$-$C_7$-cycloalkenyl, by $C_1$-$C_6$-alkoxy, by epoxy-$C_2$-$C_6$-alkoxy, by $C_2$-$C_6$-alkenyloxy or $C_2$-$C_6$-alkinyloxy, by phenoxy or heteroaryloxy, by amino, by cyano, hydroxyl or mercapto, by $C_1$-$C_6$-alkylthio, by $C_1$-$C_6$-alkylsulphinyl or $C_1$-$C_6$-alkylsulphonyl, by $C_2$-$C_6$-alkenylthio, $C_2$-$C_6$-alkenylsulphinyl or $C_2$-$C_6$-alkenylsulphonyl, by phenyl, benzyl or heteroaryl, by formyl, by $C_1$-$C_6$-alkylcarbonyl, by benzoyl, by $C_1$-$C_6$-alkylcarbonylamino, by carboxyl or $C_1$-$C_6$-alkoxycarbonyl, or by sulphamoyl, and to which one or two 3-membered to 7-membered rings can be fused which can each contain up to two double bonds and can also be aromatic or heteroaromatic,

characterized in that

a) compounds of the general formula 2

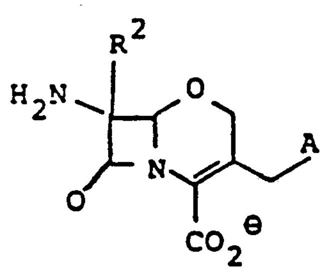

in which

A and $R^2$ have the abovementioned meaning and the amino group can also be present in the form of a reactive derivative

are reacted with a 2-(2-aminothiazol-4-yl)-2-syn-oximinoacetic acid of the general formula 3

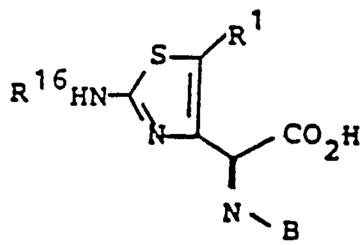

in which

B and $R^1$ have the abovementioned meaning, and

$R^{16}$ represents hydrogen or an amino-protective group

in the form of an activated derivative of this compound, and a protective group which may be present is split off,

or

b) a compound of the general formula 4

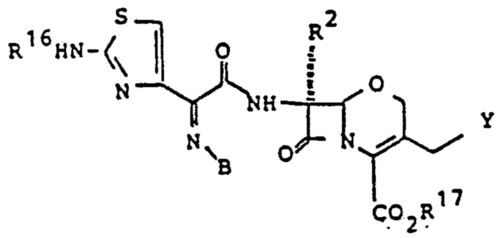

(4)

in which

$R^1$, $R^2$, $R^{16}$ and B have the abovementioned meaning, and

$R^{17}$ denotes hydrogen or an organic ammonium radical, and

Y denotes a nucleofugic leaving group, preferably chlorine, bromine or iodine, chloroacetoxy, dichloroacetoxy, mesyloxy or tosyloxy,

is reacted with pyridine or a pyridine derivative which corresponds to a pyridinium radical A mentioned in formula 1, after which, if the radical $R^{16}$ is an easily removable protective group, this group is removed hydrolytically or hydrogenolytically, thus giving the compounds of the general formula 1.

2. Process according to Claim 1, characterized in that the amino-protective group used for $R^{16}$ is a tert.-butoxycarbonyl, carbobenzoxy, trityl, formyl or chloroacetyl group.

3. Process according to Claim 1, characterized in that the reaction of the compounds of the formula 2 with carboxylic acids of the formula 3 or with their activated derivatives is carried out in a temperature range from -80°C to +80°C.

4. Process according to Claim 3, characterized in that the reaction is carried out in the presence of an inert solvent.

5. Process according to Claim 4, characterized in that the reaction is carried out in dimethylformamide.

**Revendications** pour les Etats Contractants : BE, DE, FR, IT, NL, CH, SE, GB, LI

1. Dérivés de la 1-oxadéthiacéphalosporine répondant à la formule générale 1:

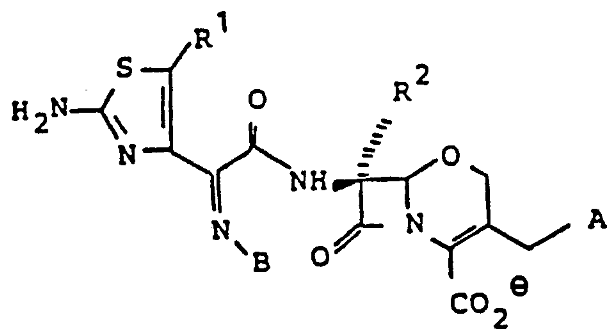

(1)

dans laquelle :

$R^1$ représente l'hydrogène, le fluor, le chlore ou le brome,

$R^2$ représente l'hydrogène ou un groupe méthoxy,

B représente un groupe alcoxy O-$R^3$ dans lequel

$R^3$ représente un groupe alkyle en $C_1$-$C_6$ ou un groupe $-(CH_2)_n(Z)_mR^4$ dans lequel :

$R^4$ représente un groupe $CO_2R^5$ dans lequel

$R^5$ représente hydrogène, un groupe alkyle en $C_1$-$C_4$, $CH_2O$-alkyle en $C_1$-$C_4$ ou un équivalent d'un métal alcalin, d'un métal alcalino-terreux, d'ammonium ou d'une base organique aminée, ou bien un groupe nitrile ou carbamoyle, et

n, m sont égaux chacun à 0 ou 1, ou bien

B représente:

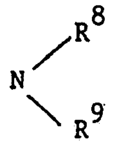 , 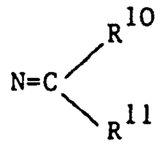 , 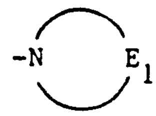 ou 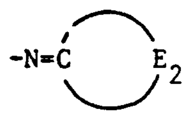

dans lesquels:

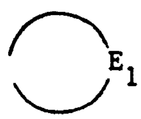

représente:

$-(CO)-(NR^{12})-(CH_2)_{n'}-$, $-(CS)-(R^{12})-(CH_2)_{n'}-$

$-(CH_2)_{m'}-R^{13}-(CH_2)_{m'}-$

$-(CO)_{m'}-(CH_2)_p-$

$-(CO)_{2-m'}-(CH_2)_{n'}-(CO)_{m'}$

$-CO-CH=CH-CO-$

$-CO-O-(CH_2)_q-$

$-CO-S-(CH_2)_q-$

$-CO-NR^{12}-CO(CH_2)_{m'}-$

$-SO_2-(CH_2)_p-$

$-CO-(CH_2)_q-SO_2-$

$-CO-NR^{12}-N=CH-$

$-(CH=CH)_2-$

$-CO-NH-N=CH-CO-$

ou

$-CO-(CH=CH)_2$

dans lesquels

$R^{12}$ a les mêmes significations que $R^8$ ou représente:

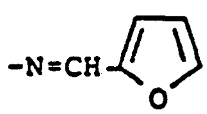

ou $-NHR^8$

$R^{13}$ représente O, S, SO, $SO_2$ ou $NR^{14}$ dans lequel

$R^{14}$ représente l'hydrogène, un groupe méthyle, éthyle, cyclopropyle, méthylsulfonyle ou furylidène-amino, et

m' est égal à 1 ou 2,

n' est égal à 2 ou 3,

p est égal à 3, 4 ou 5 et

q est égal à 2, 3 ou 4,

$E_2$ représente un groupe organique qui forme avec l'atome de carbone un noyau carbocyclique ou hétérocyclique de 4 à 7 chaînons, lequel peut contenir 1 ou 2 atomes d'oxygène, de soufre ou d'azote,

$R^8$, $R^9$, $R^{10}$ et $R^{11}$ représentent chacun, indépendmment les uns des autres, un groupe alkyle en $C_1$-$C_4$, benzyle, phényléthyle, cyclohexyle ou phényle ou un groupe hétérocyclique tel que:

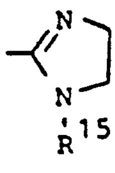, 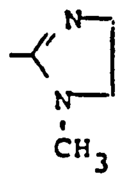, 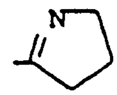, 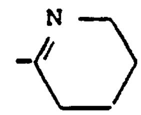

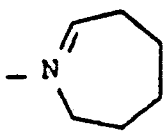, 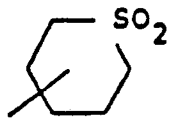, 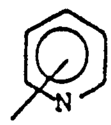, 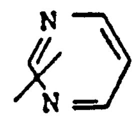,

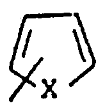, 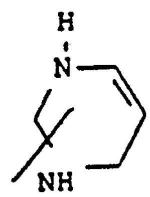, 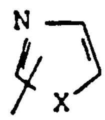, 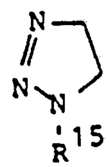,

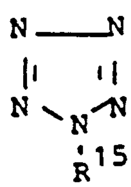, 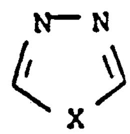,

dans lesquels:

$R^{15}$ représente l'hydrogène ou un groupe alkyle en $C_1$-$C_4$, hydroxyéthyle ou (alcoxy en $C_1$-$C_4$)-éthyle et X représente S, O, SO, $SO_2$,

Z représente un groupe 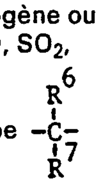

dans lequel

$R^6$ et $R^7$, qui peuvent avoir des significations identiques ou différentes, représentent chacun l'hydrogène ou un groupe alkyle en $C_1$-$C_4$ ou bien forment ensemble et avec l'atome de carbone auquel ils sont reliés un groupe méthylène ou cycloalkylidéne en $C_3$-$C_7$,

A représente un groupe pyridinium 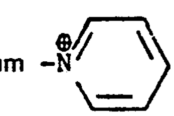

non substitué ou portant un ou plusieurs substituants identiques ou différents choisis parmi

les groupes alkyle en $C_1$-$C_6$,

les groupes cyano-alkyle en $C_1$-$C_3$, époxy-alkyle en $C_2$-$C_6$, trifluorométhyle ou pentafluoroéthyle,

les groupes hydroximino-méthyle ou (alcoxy en $C_1$-$C_4$)-iminométhyle,

les groupes alcényle en $C_2$-$C_6$,

les groupes alcynyle en $C_2$-$C_6$,

les groupes cycloalkyle en $C_3$-$C_7$ ou (cycloalkyle en $C_3$-$C_7$)-méthyle,

les groupes cycloalcényle en $C_4$-$C_7$,

les groupes alcoxy en $C_1$-$C_6$,

les groupes époxy-alcoxy en $C_2$-$C_6$,

les groupes alcényloxy en $C_2$-$C_6$ ou alcynyloxy en $C_2$-$C_6$,

les groupes phénoxy ou hétéroaryloxy,

les groupes amino,
les groupes cyano, hydroxy ou mercapto,
les groupes alkylthio en $C_1$-$C_6$, alkylsulfinyle en $C_1$-$C_6$ ou alkylsulfonyle en $C_1$-$C_6$,
les groupes alcénylthio en $C_2$-$C_6$, alcénylsulfinyle en $C_2$-$C_6$ ou alcénylsulfonyle en $C_2$-$C_6$,
les groupes phényle, benzyle ou hétéroaryle,
les groupes formyle,
les groupes (alkyle en $C_1$-$C_6$)-carbonyle,
les groupes benzoyle,
les groupes (alkyle en $C_1$-$C_6$)-carbonylamino,
les groupes carboxy ou (alcoxy en $C_1$-$C_6$)-carbonyle,
les groupes sulfamoyle, et sur lequel un ou deux cycles de 3 à 7 chaînons peuvent être condensés, ces cycles pouvant contenir chacun jusqu'à deux doubles liaisons et être de nature aromatique ou hétéroaromatique.

2. Dérivés de la 1-oxadéthiacéphalosporine selon la revendication 1, caracterisés en ce que $R^1$, $R^2$ et B ont les significations indiquées dans la revendication 1, et

A représente un groupe pyridinium 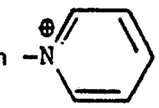

non substitué ou portant un ou plusieurs substituants identiques ou différents choisis parmi:
les groupes alkyle en $C_1$-$C_6$ eux-mêmes mono- ou poly-substitués,
les groupes hydroxy, carboxy, (alkyloxy en $C_1$-$C_6$)-carbonyle, formyle ou (alkyle en $C_1$-$C_6$)-carbonyle, carbamoyle, N-hydroxycarbamoyle, sulfo, alkyloxy en $C_1$-$C_6$, hydroxy-alkyloxy en $C_1$-$C_6$, alkylthio en $C_1$-$C_6$, alkylsulfinyle en $C_1$-$C_6$, alkylsulfonyle en $C_1$-$C_6$,alcényloxy en $C_2$-$C_6$, alcénylthio en $C_2$-$C_6$, alcénylsulfinyle en $C_2$-$C_6$ ou alcénylsulfonyle en $C_2$-$C_6$,
les groupes cyano-alkyle en $C_1$-$C_3$, epoxy-alkyle en $C_2$-$C_6$, hydroximinométhyle, (alcoxy en $C_1$-$C_4$)-iminométhyle, trifluorométhyle ou pentafluoréthyle,
les groupes alcényle en $C_2$-$C_6$ qui peuvent être substitués eux-mêmes par des groupes hydroxy, par des groupes alcynyle en $C_2$-$C_6$, cycloalkyle en $C_3$-$C_7$ ou (cycloalkyle en $C_3$-$C_7$)-méthyle, le cycle des deux substituants pouvant lui-même également être substitué par des groupes hydroxy, des halogènes, des groupes carboxy, (alkyloxy en $C_1$-$C_6$)-carbonyle ou cyano,
les groupes cycloalcényle en $C_4$-$C_7$,
les groupes alcoxy en $C_1$-$C_6$ qui peuvent eux-mêmes être substitués par des groupes hydroxy, carboxy ou (alkyloxy en $C_1$-$C_6$)-carbonyle,
les groupes époxy-alcoxy en $C_2$-$C_6$,
les groupes alcényloxy en $C_2$-$C_6$ ou alcynyloxy en $C_2$-$C_6$,
les groupes phénoxy,
les groupes amino qui peuvent eux-mêmes porter un ou deux substituants identiques ou différents choisis parmi les groupes alkyle en $C_1$-$C_6$, formyle, (alkyle en $C_1$-$C_6$)-carbonyle, (alcoxy en $C_1$-$C_6$)-carbonyle, carbamoyle, alkylsulfonyle en $C_1$-$C_6$,
les groupes cyano, hydroxy ou mercapto,
les groupes alkylthio en $C_1$-$C_6$, alkylsulfinyle en $C_1$-$C_6$ ou alkylsulfonyle en $C_1$-$C_6$ qui peuvent eux-mêmes être substitués dans la partie alkyle par des groupes hydroxy,
les groupes méthylthio, méthylsulfinyle ou méthylsulfonyle qui peuvent être substitués dans la partie méthyle par des groupes carboxy ou (alkyloxy en $C_1$-$C_6$)-carbonyle,
les groupes alcénylthio en $C_2$-$C_6$, alcénylsulfinyle en $C_2$-$C_6$ ou alcénylsulfonyle en $C_2$-$C_6$,
les groupes phényle ou benzyle qui peuvent également être substitués par des halogènes,
les groupes formyle,
les groupes (alkyle en $C_1$-$C_6$)-carbonyle qui peuvent également être substitués par des groupes hydroxy,
les groupes benzoyle ou (alkyle en $C_1$-$C_6$)-carbonylamino,
les groupes carboxy ou (alcoxy en $C_1$-$C_6$)-carbonyle,
les groupes sulfamoyle qui peuvent être monosubstitués à l'azote par un groupe (alkyle en $C_1$-$C_6$)-aminocarbonyle, et sur lequel un cycle de 3 à 7 chaînons peut être condensé, ce cycle contenant jusqu'à deux hétéroatomes du groupe formé par O, N et S et jusqu'à deux doubles liaisons et pouvant également être de nature aromatique ou hétéroaromatique, et pouvant être mono- ou poly-substitué par des groupes alkyle en $C_1$-$C_6$, cycloalkyle en $C_3$-$C_7$, alcoxy en $C_1$-$C_4$, hydroxyalkyle en $C_1$-$C_4$, des halogènes, des groupes hydroxy, oxo, hydroximino, exométhylène, carboxy, (alkyle en $C_1$-$C_6$)-oxycarbonyle, cyano, carbamoyle, sulfamoyle, amino, alkylamino en $C_1$-$C_4$ ou dialkylamino en $C_1$-$C_4$.

3. Dérivés de la 1-oxadéthiacéphalosporine selon la revendication 1, pris dans le groupe consistant en:

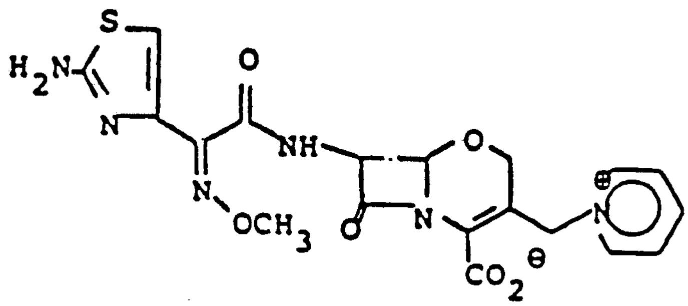

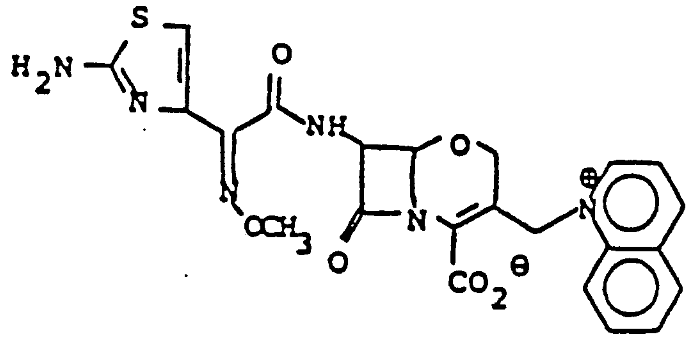

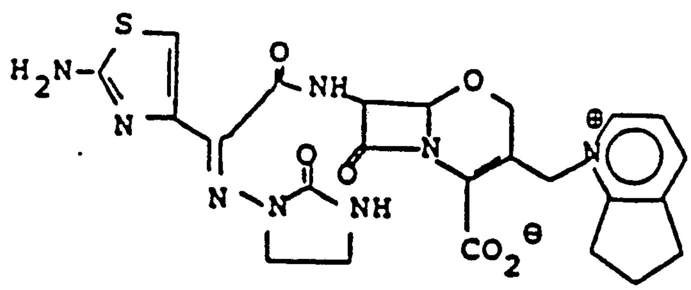

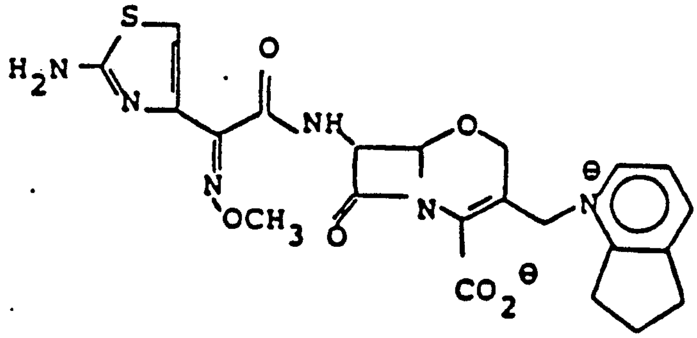

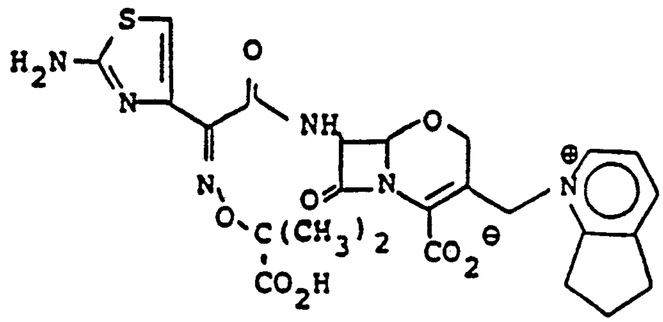

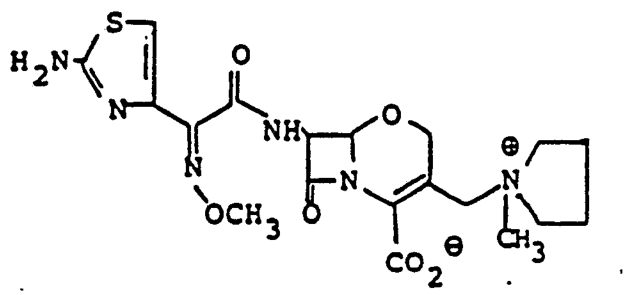

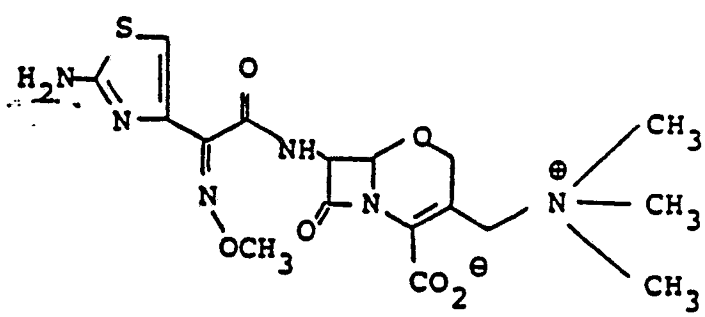

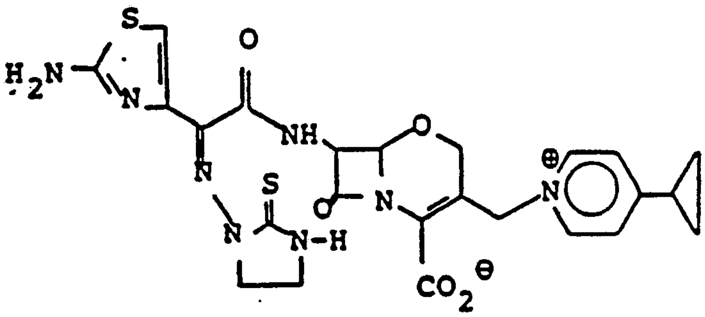

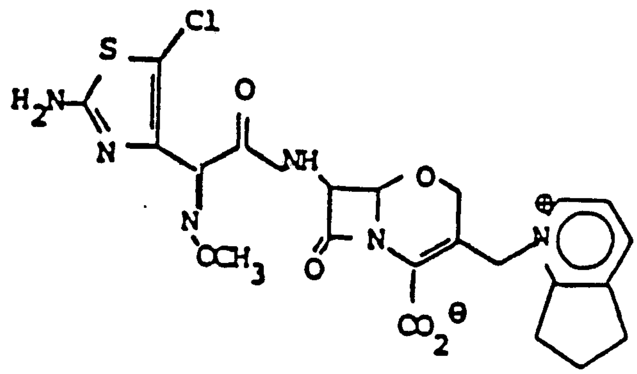

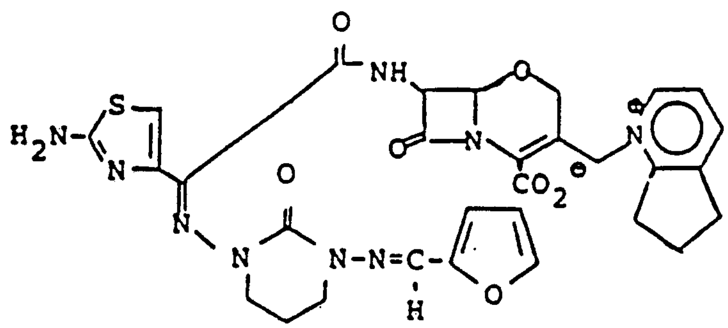

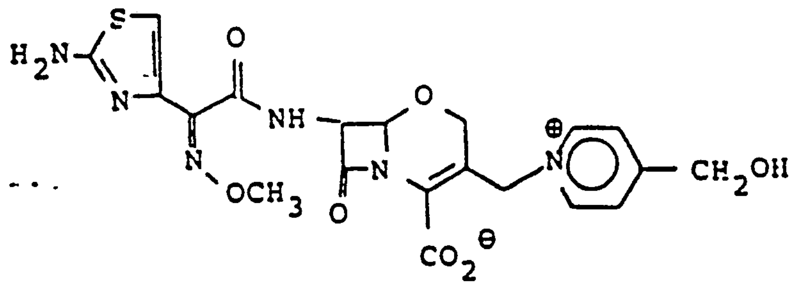

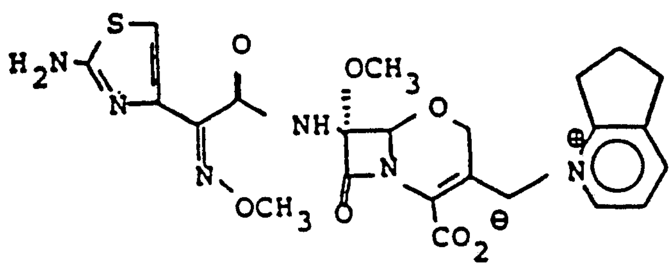

4. Procédé de préparation des dérivés de la 1-oxadéthiacéphalosporine de formule générale 1:

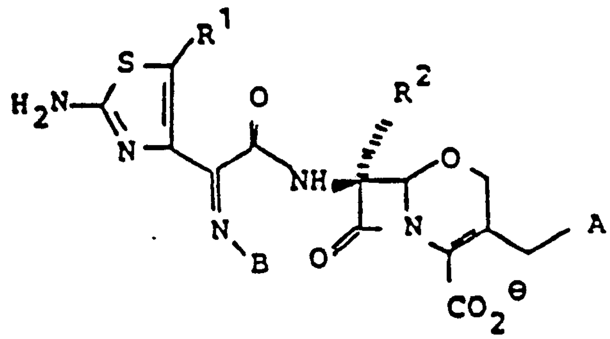

(1)

selon la revendication 1, caractérisé en ce que:

a) on fait réagir des composés de formule générale 2:

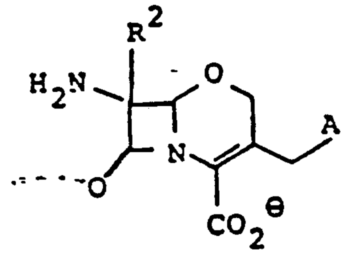

(2)

dans laquelle:

A et $R^2$ ont les significations indiquées dans la revendication 1, le groupe amino pouvant également être à l'état de dérivé réactif, avec un acide 2-(2-aminothiazole-4-yl)-2-syn-oximino-acétique de formule générale 3:

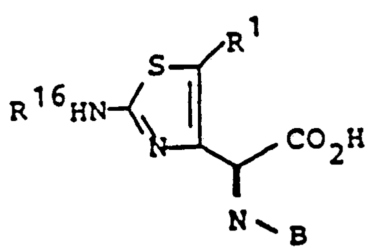

(3)

dans laquelle

B et $R^1$ ont les significations indiquées dans la revendication 1 et

$R^{16}$ représente l'hydrogène ou un groupe protecteur du groupe amino,

à l'état de dérivé activé de ce composé, et on élimine un groupe protecteur éventuellement présent, ou bien

b) on fait réagir un composé de formule générale 4:

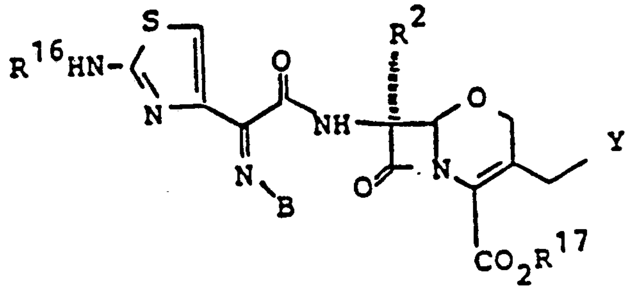

(4)

dans laquelle

$R^1$, $R^2$, $R^{16}$ et B ont les significations indiquées dans la revendication 1, et

$R^{17}$ représente l'hydrogène ou un groupe ammonium organique,

Y représente un groupe éliminable nucléofuge, de préférence le chlore, le brome ou l'iode, un groupe chloracétoxy, dichloracétoxy, méthane-sulfonyloxy ou toluène-sulfonyloxy,

avec la pyridine ou un dérivé de la pyridine correspondant à un groupe pyridinium A mentionné en référence à la formule 1, après quoi, lorsque $R^{16}$ représente un groupe protecteur facile à éliminer, on élimine ce groupe protecteur par hydrolyse ou hydrogénolyse et on obtient ainsi les composés de formule générale 1.

5. Médicament contenant au moins un composé selon l'une des revendications 1 à 3.

6. Composés de formule 1 des revendications 1 à 3 pour l'utilisation dans le traitement thérapeutique de l'organisme humain ou animal.

7. Utilisation des composés selon les revendications 1 à 3 pour la préparation de médicaments.

**Revendications** pour les Etat Contractant : AT

1. Procédé de préparation de dérivés de la 1-oxadéthiacéphalosporine de formule générale 1:

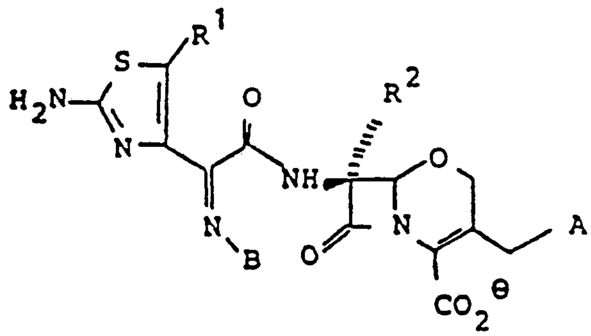

(1)

dans laquelle:

$R^1$ représente l'hydrogène, le flour, le chlore ou le brome,

$R^2$ représente l'hydrogène ou un groupe méthoxy,

B représente un groupe alcoxy O-$R^3$ dans lequel

$R^3$ représente un groupe alkyle en $C_1$-$C_6$ ou un groupe $-(CH_2)_n(Z)_mR^4$ dans lequel:

$R^4$ représente un groupe $CO_2R^5$ dans lequel

$R^5$ représente l'hydrogène, un groupe alkyle en $C_1$-$C_4$, $CH_2O$-alkyle en $C_1$-$C_4$ ou un équivalent d'un métal alcalin, d'un métal alcalino-terreux, d'ammonium ou d'une base organique aminée, ou bien un groupe nitrile ou un groupe carbamoyle, et

n et m sont égaux chacun à 0 ou 1, ou bien:

B représente:

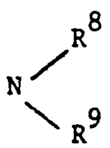 , 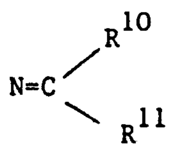 , 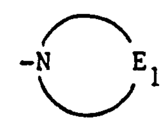 ou 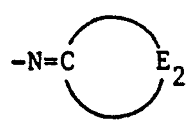

dans lequel :

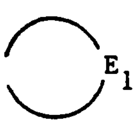

représente:

$-(CO)-(NR^{12})-(CH_2)_{n'}-$, $-(CS)-(NR^{12})-(CH_2)_{n'}-$

$-(CH_2)_{m'}-R^{13}-(CH_2)_{m'}-$

$-(CO)_{m'}-(CH_2)_p-$

$-(CO)_{2-m'}-(CH_2)_{n'}-(CO)_{m'}$

$-CO-CH=CH-CO-$

$-CO-O-(CH_2)_q-$

$-CO-S-(CH_2)_q-$

$-CO-NR^{12}-CO(CH_2)_{m'}-$

$-SO_2-(CH_2)_p-$

$-CO-(CH_2)_q-SO_2-$

$-CO-NR^{12}-N=CH-$

$-(CH=CH)_2-$

-CO-NH-N=CH-CO-
ou
-CO-(CH=CH)$_2$
dans lesquels

$R^{12}$ a les mêmes significations que $R^8$ ou représente:

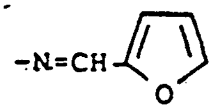

ou -$NHR^8$,

$R^{13}$ représente O, S, SO, $SO_2$ ou $NR^{14}$ dans lequel

$R^{14}$ représente l'hydrogène, un groupe méthyle, éthyle, cyclopropyle, méthylsulfonyle ou furylidène-amino, et

m' est égal à 1 ou 2,
n' est égal à 2 ou 3,
p est égal à 3, 4 ou 5 et
q est égal à 2, 3 oú 4,

$E_2$ représente un groupe organique qui forme avec l'atome de carbone un noyau carbocyclique ou hétérocyclique de 4 à 7 chaînons qui peut contenir 1 ou 2 atomes d'oxygène, de soufre ou d'azote,

$R^8$, $R^9$, $R^{10}$ et $R^{11}$ représentent chacun, indépendamment les uns des autres, un groupe alkyle en $C_1$-$C_4$, benzyle, phényléthyle, cyclohexyle ou phényle ou un radical hétérocyclique tel que:

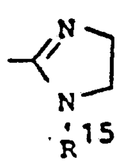 , 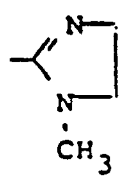 , 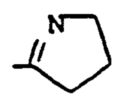 , 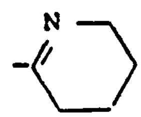 ,

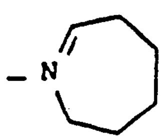 , 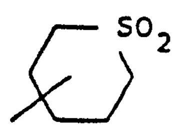 , 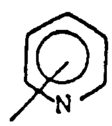 , 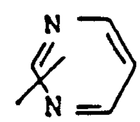 ,

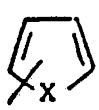 , 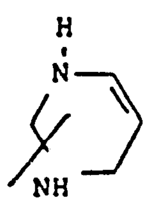 , 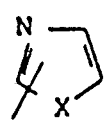 , 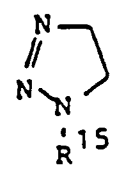 ,

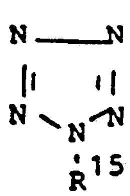 , 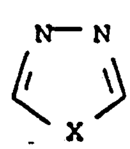 ,

dans lesquels:

$R^{15}$ représente l'hydrogène ou un groupe alkyle en $C_1$-$C_4$, hydroxyéthyle ou (alcoxy en $C_1$-$C_4$)-éthyle et
X représente S, O, SO, $SO_2$,

Z représente un groupe 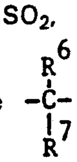 dans lequel

$R^6$ et $R^7$, qui peuvent avoir des significations identiques ou différentes, représentent chacun l'hydrogène, un

groupe alkyle en $C_1$-$C_4$ ou bien forment ensemble et avec l'atome de carbone auquel ils sont reliés un groupe méthylène ou cycloalkylidène en $C_3$-$C_7$,

A représente un groupe pyridinium 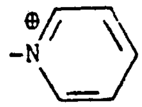

non substitué ou portant un ou plusieurs substituants identiques ou différents choisis parmi:
les groupes alkyle en $C_1$-$C_6$,
les groupes cyano-alkyle en $C_1$-$C_3$, époxy-alkyle en $C_2$-$C_6$, trifluorométhyle ou pentafluoroéthyle,
les groupes hydroximinométhyle ou (alcoxy en $C_1$-$C_4$)-iminométhyle,
les groupes alcényle en $C_2$-$C_6$,
les groupes alcynyle en $C_2$-$C_6$,
les groupes cycloalkyle en $C_3$-$C_7$ ou (cycloalkyle en $C_3$-$C_7$)-méthyle,
les groupes cycloalcényle en $C_4$-$C_7$,
les groupes alcoxy en $C_1$-$C_6$,
les groupes époxy-alcoxy en $C_2$-$C_6$,
les groupes alcényloxy en $C_2$-$C_6$ ou alcynyloxy en $C_2$-$C_6$,
les groupes phénoxy ou hétéroaryloxy,
les groupes amino,
les groupes cyano, hydroxy ou mercapto,
les groupes alkylthio en $C_1$-$C_6$, alkylsulfinyle en $C_1$-$C_6$ ou alkylsulfonyle en $C_1$-$C_6$,
les groupes alcénylthio en $C_2$-$C_6$, alcénylsulfinyle en $C_2$-$C_6$ ou alcénylsulfonyle en $C_2$-$C_6$,
les groupes phényle, benzyle ou hétéroaryle,
les groupes formyle,
les groupes (alkyle en $C_1$-$C_6$)-carbonyle,
les groupes benzoyle,
les groupes (alkyle en $C_1$-$C_6$)-carbonylamino,
les groupes carboxy ou (alcoxy en $C_1$-$C_6$)-carbonyle,
les groupes sulfamoyle, et sur lequel un ou deux cycles de 3 à 7 chaînons peuvent être condensés, ces cycles pouvant contenir chacun jusqu'à deux doubles liaisons et également être de nature aromatique ou hétéroaromatique,

caractérisé en ce que:

a) on fait réagir des composés de formule générale 2:

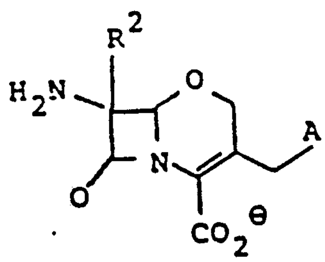

(2)

dans laquelle:

A et $R^2$ ont les significations indiquées ci-dessus, le groupe amino pouvant également être à l'etat de dérivé réactif, avec un acide 2-(2-aminothiazole-4-yl)-2-syn-oximino-acétique de formule générale 3:

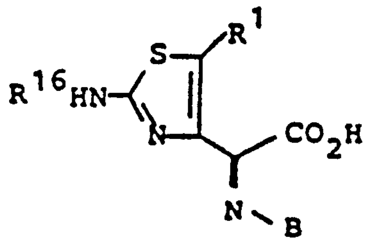

(3)

dans laquelle:

B et $R^1$ ont les significations indiquées ci-dessus, et

$R^{16}$ représente l'hydrogène ou un groupe protecteur du groupe amino, à l'état de dérivé activé de ce composé, et on élimine un groupe protecteur éventuellement présent, ou bien

b) on fait réagir un composé de formule générale 4:

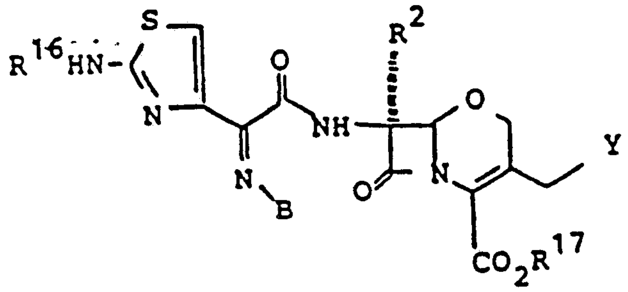

(4)

dans laquelle:

$R^1$, $R^2$, $R^{16}$ et B ont les significations indiquées ci-dessus, et

$R^{17}$ représente l'hydrogène ou un groupe ammonium organique,

Y représente un groupe éliminable nucléofuge, de préférence le chlore, le brome ou l'iode, un groupe chloracétoxy, dichloracétoxy, méthane-sulfonyloxy ou toluène-sulfonyloxy,

avec la pyridine ou un dérivé de pyridine correspondant à un groupe pyridinium A mentionné en référence à la formule 1, après quoi, lorsque $R^{16}$ représente un groupe protecteur facile à éliminer, on élimine ce groupe par hydrolyse ou hydrogénolyse et on obtient ainsi les composés de formule générale 1.

2. Procédé selon la revendication 1, caractérisé en ce que le groupe protecteur du groupe amino représente par $R^{16}$ est un groupe tert-butoxycarbonyle, carbobenzoxy, trityle, formyle ou chloracétyle.

3. Procédé selon la revendication 1, caractérisé en ce que l'on effectue la réaction entre les composés de formule 2 et les acides carboxyliques de formule 3 ou leurs dérivés activés dans un intervalle de température de -80 à +80°C.

4. Procédé selon la revendication 3, caractérisé en ce que l'on effectue la réaction en présence d'un solvant inerte.

5. Procédé selon la revendication 4, caractérisé en ce que l'on effectue la réaction dans le diméthylformamide.